(19) Europäisches Patentamt
European Patent Office
Office européen des brevets

(11) EP 1 007 541 B1

(12) EUROPEAN PATENT SPECIFICATION

(45) Date of publication and mention of the grant of the patent:
**23.08.2006 Bulletin 2006/34**

(21) Application number: **98941652.4**

(22) Date of filing: **28.08.1998**

(51) Int Cl.:
***C07K 1/04*** *(2006.01)* ***C07K 14/47*** *(2006.01)*
***G01N 33/68*** *(2006.01)*

(86) International application number:
**PCT/IB1998/001469**

(87) International publication number:
**WO 1999/011654 (11.03.1999 Gazette 1999/10)**

(54) **RANDOMLY GENERATED GLYCOPEPTIDE COMBINATORIAL LIBRARIES**
ZUFÄLLIGVERTEILT ERZEUGTE KOMBINATORISCHE PEPTIDBIBLIOTHEKEN
BANQUES COMBINATOIRES DE GLYCOPEPTIDES GENERES DE MANIERE ALEATOIRE

(84) Designated Contracting States:
**AT BE CH CY DE DK ES FI FR GB GR IE IT LI LU MC NL PT SE**

(30) Priority: **28.08.1997 US 56240 P**

(43) Date of publication of application:
**14.06.2000 Bulletin 2000/24**

(73) Proprietor: **BIOMIRA, INC.**
**Edmonton,**
**Alberta T6N 1H1 (CA)**

(72) Inventors:
- **KOGANTY, Rao, R.**
  **Edmonton, Alberta T6J 3R3 (CA)**
- **QIU, Dongxu**
  **Edmonton, Alberta T6L 6T3 (CA)**
- **GANDHI, Sham**
  **Edmonton, Alberta T6L 2T5 (CA)**

(74) Representative: **Maschio, Antonio et al**
**D Young & Co**
**120 Holborn**
**London EC1N 2DY (GB)**

(56) References cited:
**WO-A-95/10296** **WO-A-95/18971**

- **A SCHLEYER ET AL.: "Direct solid-phase glycosylations of peptide templates on a novel PEG-based resin " ANGEWANDTE CHEMIE INTERNATIONAL EDITION., vol. 36, no. 18, 2 October 1997, pages 1976-1978, XP002092747 WEINHEIM DE**
- **CHEMICAL ABSTRACTS, vol. 125, no. 21, 18 November 1996 Columbus, Ohio, US; abstract no. 276512, K FRISCHE ET AL.: "Multiple column synthesis of a library of T-cell stimulating Tn-antigenic glycopeptide analogs for the molecular characterization of T-cell glycan specificity" XP002092749 & J. PEPT. SCI. , vol. 2, no. 4, 1996, pages 212-222,**
- **File Medline, abstract 96121420, 1996 XP002092748 & X LIU ET AL.: "Structurally defined syntetic cancer vaccines; analysis of the structure, glycosylation and recognition of cancer associated mucin, MUC-1 derived peptides" GLYCOCONJUGATE JOURNAL, vol. 12, no. 5, October 1995, pages 607-617,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 007 541 B1

**Description**

## BACKGROUND OF THE INVENTION

The present invention relates to a method for generating a combinatorial library of glycopeptides, and to glycopeptide libraries produced by the method. More particularly, it relates to a method for generating a combinatorial library of a cancer-associated mucin.

Glycopeptides are a broad class of organic compounds that are important in diverse biochemical processes, including cell growth regulation, binding of pathogens to cells, intercellular communication, and metastasis. Biosynthetically, the carbohydrates of glycoproteins are attached (co- or posttranslationally) by glycosyltransferase enzymes, each enzyme being specific for a particular monosaccharide unit and linkage type. Mono- and oligosaccharides are transferred to proteins in the endoplasmic reticulum and linked to the $NH_2$ group on the side chain of an asparagine residue of the protein, to form N-linked oligosaccharides. Mono- and oligosaccharides also may be linked to the OH group on the side chain of a serine, threonine, or hydroxylysine residue, to form O-linked oligosaccharides.

Different glycoforms of the same protein are regularly found, which differ in the carbohydrate structures that are attached. The glycoforms vary in properties such as protease stability, affinity to receptors, and pharmacokinetic profile. To study the influence of glycosylation patterns on the properties of a glycopeptide, and to identify compounds useful in diagnosis and/or therapy, it is necessary to have access to several different glycoforms. The most generally applied approach for obtaining defined glycopeptide fragments is chemical synthesis using glycosylated amino acids, although enzymatic glycosylation using glycosyltransferases has also been used.

Chemical synthesis of serine and threonine with large O-linked carbohydrate structures as building blocks for glycopeptide synthesis is much more difficult than synthesis of either oligopeptides or oligosaccharides. For example, a number of mono-, di- and trisaccharides have been identified on the core of the carcinoma-associated MUC1. Chemical synthesis of the α-O-linked N-acetylgalactosamine-based mucin-type glycopeptides depends on the accessibility to large amounts of O-glycosylated amino acids. Sequential glycosylations of serine and threonine for Fmoc-based glycopeptide synthesis involves a complex manipulation of the selectivities of base-sensitive protecting groups. Fmoc-protected serine and threonine are among the more highly sensitive and hindered aglycons used in glycosylation reactions. It is virtually impossible to synthesize chemically all possible combinations of glycopeptides and test them against a natural anti-mucin antibody or polyclonal serum.

Combinatorial methods have gained great interest as a method of finding desirable compounds. These methods involve creating libraries of related compounds. Interaction of an antigen with the library is then measured, in order to assess whether one or more compounds in the library recognizes the antigen. The use of combinatorial chemistry to synthesize large numbers of molecules, either as individual compounds or as mixtures, is considered to be one of the frontiers of organic chemistry applied to drug discovery.

To date, combinatorial chemistry has been applied to the generation of peptide and oligonucleotide libraries, where the well established chemistry of amide bond and phosphodiester bond formation led to rapid progress. Combinatorial chemistry has not been applied, however, to the generation of glycopeptide libraries. Glycosylation of the amino group on the side chain of an asparagine residue of the protein, to form N-linked oligosaccharides, may occur at any one of the three or four hydroxyl groups on the saccharide. For both N-linked and O-linked oligosaccharides, a new stereocenter is formed on glycosylation. This results in either an α- or β-glycosidic linkage, which are usually axial and equatorial, respectively.

WO-A-95/18971 discloses methods for the solid phase synthesis of glycoconjugates including glycopeptides. This document discloses libraries consisting of glycopeptides with differently glycosylated species including peptide libraries containing peptides being multiply glycosylated in particular by reacting at least two glycosyl donors with peptides having more than one glycosylation site. WO-A-95/10296 is directed also to combinatorial libraries of glycopeptides for the identification of cell adhesion inhibitors. The generation of the different species forming the combinatorial library is perfomed by glycosylating peptide scaffolds.

A need therefore exists for a method of generating a combinatorial library of glycopeptides. Such a library could be used to screen for biological activity of different glycoforms within the library.

## SUMMARY OF THE INVENTION

It is therefore an object of the present invention to provide a combinatorial method for the generation of glycopeptide libraries.

It is another object of the invention to provide a method of identifying glycopeptides that have a defined biological activity by screening a random library of glycopeptides for the biological activity.

It is yet another object of the invention to provide a method of generating a combinatorial library of mucins.

These and other objects of the invention are achieved by a method of generating a library peptides or peptide-like structures that have at least one glycosylation site comprising (a) randomly glycosylating a core having at least one glycosylation site with at least one glycosyl donor, optionally blocking unreacted glycosylation sites on the glycosylated cores and optionally selectively removing one or more protecting groups on the carbohydrate groups introduced at the first level; whereby a first level library of glycosylated cores is created; and then (b) optionally randomly glycosylating said first level library of glycosylated cores, or a combination of first level libraries of glycosylated cores with at least one glycosyl donor, and optionally selectively removing one or more designated protecting groups on the carbohydrate groups introduced at the second level; whereby a second level library of glycosylated cores is created. The method may further comprise randomly glycosylating the second level library of glycosylated cores, or a combination of second level or first and second level libraries of glycosylated cores, with at least one glycosyl donor, and optionally selectively removing one or more designated protecting groups on the carbohydrate groups introduced at the third level; whereby a third level library of glycosylated cores is created; and optionally repeating the foregoing step to produce fourth and higher level libraries of increased diversity.

In a preferred embodiment, the randomly-generated glycopeptide library comprises carcinoma-associated mucins or structures associated with adhesion ligands for bacterial receptors that are expressed on human cell surface antigens. Components of the library can be screened for drug-like, competitive inhibitory, immunostimulatory or antibody-like activity.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

## BRIEF DESCRIPTION OF THE DRAWINGS

Figure 1 shows a linear platform for combinatorial library synthesis according to the present invention.

Figure 2 (a cyclic MUC1 peptide) is an example of a cyclic platform for combinatorial library synthesis according to the present invention.

Figure 3 shows a simple cyclic peptide and solubilized version which contains a lipid chain.

Figure 4 shows a platform with unnatural glycosylation sites.

Figure 5 is an example of a hybrid platform which does not include peptide linkages.

Figure 6 (an example of a cyclic peptide for random glycosylations solubility of such peptides may be enhanced by hydrophobic groups) is an example of a cyclic peptide for random glycosylations, in which solubility is enhanced by introduction of hydrophobic groups.

Figure 7 shows carbohydrate structures found on cancer mucins.

Figure 8 (functional demonstration of glycopeptide library with well characterized monoclonal antibodies) is a bar graph of the results of screening of a GSTA library.

## DETAILED DESCRIPTION OF THE PREFERRED EMBODIMENTS

In accordance with the present invention, it is possible to generate large glycopeptide libraries by random glycosylation of a selected peptide or peptide-like structure that has at least one glycosylation site. Random glycosylation of a peptide or peptide-like structure with more than one glycosylation site yields a large library of all possible combinations of glycosylation since each site of glycosylation is unique for a given sequence. The random libraries provide a fast and efficient way of screening for drug-like, competitive inhibitory, immunostimulatory, antibody-like and other biological activities. The size of the random library of glycopeptides depends on the extent of glycosylation in terms of number of sites and the variety of carbohydrate structures that are added.

The "platform" or core used as the basis for the glycopeptide library is any peptide or peptide-like structure, including unnatural, synthetic structures, and may contain tandem repeats. The platform includes one or more glycosylation sites, which may be natural or unnatural. A platform with unnatural glycosylation sites is shown in Figure 4. Preferably there are from 2 to 5 glycosylation sites on the peptide or tandem repeat, and more preferably 2 or 3 glycosylation sites. In a preferred embodiment, each glycosylation site on a platform is unique and distinguishable from other sites due to distinct structural features in the vicinity of the site.

Where the platform is a peptide-like structure, it does not necessarily contain peptide linkages, but may comprise any structure with glycosylation sites to which carbohydrate structures may be attached. For example, a platform may be a "hybrid" platform comprising a non-peptide polymer or even a chain of carbon atoms to which natural amino acid side chains with natural glycosylation sites are attached. An example of a hybrid platform in shown in Figure 5.

The glycosylation sites provide hydroxy functions for O-glycosylation and/or natural carboxy or carboxamido

functional groups for N-glycosylation. Preferred glycosylation sites include one or more of serine, threonine and hydroxylysine, the hydroxyl group of which provides O-glycosylation sites, or asparagine, the amino group of which provides an N-glycosylation site.

Glycosylation sites my be of either d- or 1-optical configuration, although it is preferable that the glycosylation sites consist entirely of d-optical configuration. It is more particularly preferred that the entire platform be constructed of d-amino acids.

The platform may be linear (Figure 1) or cyclic (Figure 2), and may carry UV-active or fluorescent labels to aid in detection during a process of screening a glycopeptide library produced using the platform. Hydrophobic amino acids preferably are incorporated in the platform, as shown in Figure 6, in order to increase the solubility of the platform in the organic solvents used to promote glycosylation reactions. In a preferred embodiment, glycosylation sites are spaced, singly or in clusters, between sequences that include hydrophobic amino acids such as alanine, phenylalanine, valine, leucine and isoleucine or unnatural hydrophobic amino acids. Lipid chains also can be incorporated into the platform to aid in the coating of microtiter plates, as shown in Figure 3.

The peptide of the platform may be isolated or synthesized by any known method. The peptide may have free groups at the glycosylation binding sites, or it may be partially blocked, so that only certain glycosylation sites are available. The blocking groups may be introduced selectively during the synthesis of the platform, or may be introduced at any time during a series of glycosylation reactions on the platform. The blocking sites also may be selectively removed during any step of then process.

Carbohydrate structures are randomly introduced or arranged on the platform to generate a diverse glycopeptide library. Carbohydrate structures may include or be derived from glycoproteins, proteoglycans and glycolipids found on both normal and malignant cells. Carbohydrate structures may be pre-designed and restricted in number for more efficient screening of the resulting combinatorial library for specific purposes. Carbohydrate structures may be unnatural, i.e., a random combination of monosaccharides linked together with a mix of $\alpha$- and $\beta$-linkages.

Glycosyl (carbohydrate) donors are selected based on knowledge of the carbohydrate moieties contained in a glycopeptide of interest. One preferred group of carbohydrate structures includes those structures known to be adhesion ligands for bacterial receptors that are expressed on human cell surface antigens. Another preferred group of carbohydrate structures include those known to be associated with malignant cell antigens.

For example, N-acetylgalactosamine O-linked to serine or threonine is known as carcinoma-associated Tn antigen, while the disaccharide $\beta$Gal(1-3)$\alpha$GalNAc O-linked to serine or threonine in mucins is commonly known as carcinoma-associated Thomsen-Friedenreich (TF) antigen. The disaccharide is also found on the cell surface of chronic and acute myelogenous leukemic leukocytes. Sialylated versions of TF, particularly $\alpha$2-6-TF, have been found on the mucins expressed by human breast cancer cell lines. The structure and synthesis of TF family glycosyl donors is described by Qiu *et al.*, *Tetrahedron Letters*, 37:595-585 (1996). (The contents of this document, and all other documents specifically cited herein, are incorporated in this disclosure in their entirety by reference.) Examples of carbohydrate structures found on cancer mucins are found in Figure 7.

Thus, when the desired glycopeptides are carcinoma-associated mucins, glycosyl donors used to glycosylate the core peptide include galactosamine, N-acetylgalactosamine, and sialyl. When generating a combinatorial library for glycopeptides other than Tn and TF glycopeptides, other glycosyl donors are employed.

The number of glycosyl donors at each level preferably is at least 2, and more preferably at least 3. Typically, more donors are used at levels beyond first level than are used in generating the first level library. It may be preferred in some instances to limit the size of a library by limiting the number of donors at each level to less than 5. Synthesis of glycosyl donors is well known in the art.

Glycosyl donors can be designed, in accordance with the present invention, to yield only particular carbohydrate structures of interest. In this regard, a glycosyl donor may be designed with protecting groups, such as 4,6-benzylidene, to favor formation of a particular carbohydrate structure. See, *e.g.*, Qiu *et al.*, *supra,* and Yule *et al.,* Tetrahedron Letters, 36:6839-6842 (1995), and Broddefalk *et al.,* Tetrahedron Letters, 17:3011-3014 (1996). The glycosyl donors, as well as glycosylation sequence, may be selected based on a predetermined assessment of the nature and number of established carbohydrate structures for a selected glycopeptide.

Glycosyl donors are reacted with a core peptide according to methods well known in the art. See, *e.g.,* Qiu *et al.* and Yule *et al.*, *supra*, as well as Kunz, Angew. *Chem. Int. Ed. Engl.* 26:294-308 (1987) and Garg *et al., Advances in Carbohydrate Chemistry and Biochemistry,* 50:277-310 (1994). A first level library of a desired glycopeptide is created by primary glycosylation of the peptide with a single glycosyl donor or a mixture of donors. Reaction of a core peptide with a glycosyl donor, or mixture of donors, results in a library of randomly glycosylated glycopeptides. A first level library can be used in a screening process, as described below, or can form the basis for generating higher level libraries.

A second level library is created by reacting one or more first level libraries with one or more further glycosyl donors. Prior to further reaction, unreacted glycosylation sites on the peptides may be blocked, e.g., by acetylation, in order to prevent these glycoforms from being eliminated from the library by being converted into different glycoforms. Following purification, the protecting groups of the carbohydrate structures on the glycoforms are selectively removed

to create additional glycosylation sites on the existing carbohydrate structures. Random glycosylation with these additional donors further extends existing carbohydrate structures, thereby to create more complex glycopeptide structures. Higher level libraries are similarly created by reacting one or more second level or higher libraries with one or more further glycosyl donors.

The total number of possible glycoforms contained in a combinatorial library of glycopeptides according to the invention can be calculated by the following formula:

$$\text{\# of glycoforms} = (x + 1)^n$$

where x is the number of glycosyl donors used and n is the number of glycosylation sites. For example, the following table shows the number of glycoforms that are obtained when up to five glycosyl donors and up to five glycosylation sites are used:

Table 1.

| # of sites | carbohydrate structures (x) | | | | |
|---|---|---|---|---|---|
| | 1 | 2 | 3 | 4 | 5 |
| 1 | 2 | 3 | 4 | 5 | 6 |
| 2 | 4 | 9 | 16 | 25 | 36 |
| 3 | 8 | 27 | 64 | 125 | 216 |
| 4 | 16 | 81 | 256 | 625 | 1296 |
| 5 | 32 | 243 | 1024 | 3125 | 7776 |

It is possible to selectively control the size and complexity of the glycopeptide libraries in a number of ways. The size and complexity of a random library of glycopeptides depend on the extent of glycosylation, which in turn depends on the number of glycosylation sites on the core peptide and on the number of glycosyl units that are added. Libraries with various combinations of glycoforms can be achieved by mixing lower level libraries before glycosylation, by mixing donors at each level, and by using mixed lower level libraries in combination with mixed donors. Sites of further glycosylation can be controlled by protecting unreacted glycosylation sites on the peptide, thereby preserving those structures in the library.

The glycopeptide libraries can be used to screen for biologically active compounds. By screening a number of libraries, each with different combinations of carbohydrate structures on the core peptide, it is possible to identify which structures have drug-like, competitive inhibitory; immunostimulatory, antibody-like, and many other biological activities. A library according to the present invention can be screened for compounds that have anti-bacterial activity, including compounds that competitively inhibit bacterial adhesion to a host cell. The library also may be screened for compounds that have anti-viral activity. In a preferred embodiment libraries are generated and screened to develop highly sensitive diagnostic antibodies and antigens for the detection and immunotherapy of cancers.

Methods of screening for biological activities using combinatorial libraries are known in the art. U.S. Patent Nos. 5,510,240 and 5,541,061 provide examples of methods of screening combinatorial libraries, but any suitable method of screening for biological activity using combinatorial libraries may be used in accordance with the present invention. For example, an ELISA of various antibodies binding either Ovine Submaxillary Mucin (OSM) or CA27.29 as the solid phase may be used in an inhibition format with components from the libraries as inhibitors.

In a preferred embodiment, the peptide is derived from a cancer-associated mucin, and is in particular a MUC1 core protein. The MUC1 tandem repeat derived sequence GVTSAPDTRPAPGSTA, contains five O-glycosylation sites, two serines and three threonines, and is an example of a peptide that can be glycosylated according to the present invention to create a glycopeptide library. If all possible glycosylation sites in a tandem repeat are used only once in primary glycosylation with N-acetylgalactosamine (Tn antigen), five different monoglycosylated tandem repeats result, but if glycosylation is randomized between 0 and 5 sites, there, are 32 different combinations of glycosylated tandem repeats. If 0 to 5 sialic acids are then randomly added at the 6-position of the existing N-acetylgalactosamines, the possible number of glycoforms increases to 243. These will carry only combinations and varied numbers of Tn and STn. If another donor is added at each glycosylation, *e.g.,* TF along with the first and GlcNAc along with the second, a total of 16807 glycosylation variants of MUC1 tandem repeat will be produced. This library will constitute more than 90% of all truncated versions (core structures) that may be associated with cancerous MUC1 mucin. These are useful as vaccine components.

A simpler tetrapeptide, GSTA, has only two 0-glycosylation sites and is useful in demonstrating the method according to the invention, as shown in the following examples. The protecting groups on the glycosyl donors and the sequence of glycosylation reactions are designed to yield only the established carbohydrate structures possible in mucin biosynthesis. The "rules" observed by the glycosyltransferases in creating carbohydrate structures on mucins are shown in Table 2, wherein GalNAc is N-acetylgalactosamine, GlcNAc is N-acetylglucosamine, and Gal is galactose. Sialic acid is another name for N-acetylneuraminic acid. It is notable that the N-acetylgalactosamine-based glycosyl donors form only alpha linkages with serine and threonine hydroxyls.

The library of GSTA glycopeptides modelled on naturally-existing mucins, is small enough that the components can be characterized by mass spectrometry. It is therefore very useful in gaining a precise understanding of glycosylation patterns of the MUC1 core protein, which is necessary in order to design effective therapeutic vaccines and diagnostic tools.

Table 2.

| Donor | Linkage | Site | Example |
|---|---|---|---|
| GalNAc | alpha | serine, threonine and 3-O GalNac | Tn, Core 6 |
| GlcNAc | beta | 6-O GalNac, 3-O Gal | F1 alpha |
| Sialic acid | alpha | 6-O GalNAc, 3-O Gal | STn, STF |
| Gal | beta | 3-O GalNAc, 4-O GlcNAc | TF, F1 alpha |

The following examples illustrate generation of a library of GSTA glycopeptides according to the present invention, but do not limit the scope of the invention in any way. Further aspects and variations of the invention, based on the disclosure above and the following examples, will be apparent to the person of ordinary skill in the art.

**EXAMPLES**

Example 1: Synthesis of protected peptides of GSTA

GSTA is a four amino acid residue of MUCl, which has two unique sites for glycosylation, the serine residue (S) and the threonine residue (T). It is manually synthesized in solution with N-terminal Fmoc and C-terminal benzyl, with serine and threonine hydroxyls free.

Glycosyl donors N-acetylgalactosamine (Tn antigen) and βGal(1-3)αGalNAc (carcinoma associated Thomsen-Friedenreich, or TF antigen) are protected with 4,6-benzylidenyl protecting groups. Synthesis of protected peptides of GSTA is shown in Reaction Scheme I.

**Preparation of compound 1**

A mixture of L-alanine, 20 g, 0.22 mol and toluene-4-sulphonic acid, 53 g, 0.28 mol in 100 mL of benzyl alcohol and 150 ml of benzene were refluxed overnight using a Dean stark apparatus. Benzene was removed *in vacuo* and 250 ml of ether was added to give compound **1**, a solid, 75 g, 78%. [α] D-5.2 (c0.25, MeOH); $^1$H-NMR (300 MHz, CDC13), 8=8.25 (m, 3 H, TSOH, 2 $NH_2$), 7.00-7.70 (m, 9 H, Ar-H), 5.08, 4.98 (2 d, 2 H, J=12.5 Hz, $CH_2$), 4.05 (dd, I H, J=7.5, 15.0 Hz, Ala-(x-H), 2.30 (s, 3 H, $PhCH_3$), 1.43 (d, 3 H, J=7.0 Hz, $CH_3$).

**Preparation of compound 3**

To a solution of N-Boc-L-threonine 2, 10 g, 45.6 mmol in 80 mL of dry THF was added 3.5 mL of N-methyl morpholine and 4.5 mL of i-butyl chloroformate at -20°C under nitrogen. After stirring at -20°C for 5 min., a solution of compound 1, 15 g, 57 mmol and 3.5 ml of N-methyl morpholine in 40 ml of dry THF and 5 ml of dry DMF was added. After stirring at -20°C for 30 min., 10 mL of methanol was added and the solvent was removed in vacuo. The residue was purified by silica gel column using ethyl acetate/hexane (1:1) to give compound **3**, a white powder after freeze drying from dioxane, 12.5 g, 71%. [α]D -39.2 (cO. 25, MeOH); $^1$H-NMR (300 MHz; $CDCl_3$), 6=7.30-7.50 (m, 5 H, Ar-H), 7.10 (d, 1 H, J=7.0 Hz, NH), 5.56 (d, 1 H, J=8.0 Hz, NH), 5.20, 5.14 (2 d, 2 H, J= 12.0 Hz, $CH_2Ph$) , 4.60 (m, 1 H, Ala-α-H), 4.30 (m, 1 H, Thr-β-H), 4.14 (dd, 1 H, J=2.0, 7.5 Hz, Thr-(x-H), 3.46 (m, 1 H, OH), 1.45 (s, 9 H, 3 $CH_3$), 1.41 (d, 3 H, J=7.5 Hz, CH3), 1.18 (d, 3 H, J=6.5 Hz, $CH_3$)

**Preparation of compound 4**

A solution of compound 3, 8.7 g, in 50 mL of formic acid was stirred at room temperature for 5 hours. Formic acid was removed in vacuo and ethyl acetate was added to the residue to give compound **4**, a solid, 5.3 g, 71%. [α] D-66.8 (co.25, MeOH) ; $^1$H-NMR (300 MHz, $CDCl_3$+$CD_3OD$), δ=8.42 (s, 1 H, HCOOH), 7.30-7.50 (m, 5 H, Ar-H), 7.10 (d, 1 H, J=7.0 Hz, NH), 5.56 (d, I H, J=8.0 Hz, NH), 5.17, 5.09 (2 d, 2 H, J=12.0 Hz, $CH_2Ph$), 4.50 (dd, 1 H, J=7.5, 15.0 Hz, Ala-(α-H), 3.89 (m, 1 H, Thr-α-H), 3.48 (d, 1H, J=7.5 Hz, Thr- (X-H), 1.41 (d, 3 H, J=7.5 Hz, $CH_3$), 1.23 (d, 3 H, J=6.5 Hz, $CH_3$); ES-MS. Calc. for $C_{14}H_{20}O_4N_2$, 280.3; Found, 279.4.

**Preparation of compound 5**

A solution of N-Fmoc-L-glycine, 100 g, 0.336 mol and DCC, 139 g, 0.67 mol in 1000 mL of dry ethyl acetate was stirred at 5°C for 15 min. A solution of N-hydroxysuccinimide, 138.8 g, 0.67 mol in 1000 mL of dry ethyl acetate was added. After stirring at 5°C for 2 hours, the solid was filtered and washed with ethyl acetate (3x50 mL). The solvent was removed *in vacuo* and the residue was purified by crystallization from hexane and ethyl acetate to give compound 5, a solid, 83.2 g, 63%. [α]D+6.0(c0.25, MeOH); $^1$H-NMR (300 MHz, $CDCl_3$), δ=7.30-7.80 (m, 8 H, Ar-H), 5.42 (t, 1 H, J=5.5 Hz, NH), 4.43(d, 2 H, J=7.0 Hz, $CH_2$ on Fmoc), 4.37(d, 2 H, J=6.0 Hz, Ala-(α-H), 4.23(t, 1H, J=7.0 Hz, CH on Fmoc), 2.84 (s, 4 H, 2 $CH_2$).

**Preparation of compound 7**

To a solution of L-serine, 20 g, 0.18 mol and sodium bicarbonate, 16 g in 150 mL of water was dropped compound 5, 56 g, 0. 142 mol in 600 ml of DME. The mixture was stirred at room temperature overnight. DME was removed *in vacuo* and the 5% of aqueous HCI was added to adjust pH=2. The water solution was extracted with ethyl acetate (3x200 mL). The organic layer was washed with water and dried over Na2SO4. The solvent was removed in *vacuo* and the residue was purified by silica gel column using ethyl acetate/acetic acid (9:1) to give compound 7, a white powder after freeze drying from dioxane, 35 g, 62%. [α]D+9.6 (cO.50, MeOH); $^1$H-NMR (300 MHz, $CDCl_3$+$CD_3OD$), δ=7.30-7.80 (m, 8 H, Ar-H), 4.61 (t, 1 H, J=4.0 Hz, ser-α-H), 4.45 (d, 2 H, J=7.0 Hz, $CH_2$ on Fmoc), 4.30 (t, 1 H, J=7.0 Hz, CH on Fmoc), 4.03 (dd, 1 H, J=4.0, 11.5 Hz, ser-α-H), 3.90-4.08 (m, 3H) . ES-MS: Calc. for $C_{20}H_{20}O_7N_2$, 384.3; Found, 384.3.

**Preparation of compound 8**

To a solution of compound 7, 1.7 g, 4.25 mmol in 20 mL of dry THF was added 0.44 mL of N-methylmopholine and 0.52 mL of i-butylchloroformate at -20°C under nitrogen. After stirring at -20°C for 5 min., a solution of compound 3, 1.12 g, 4 mmol and 0.44 mL of N-methylmorpholine in 10 mL of dry THF and 5 mL of dry DMF was dropped in 5 min. After stirring at -20°C for 30 min., 5 mL of methanol was added and the solvent was removed in vacuo. The residue was purified by silica gel column using hexane/ethyl acetate/methanol (10:10:1) to give compound 8, a white powder after freeze drying from dioxane, 1.7 g, 66%. [α]D-36.5(c0.2, MEOH: $CHCl_3$=4:1) ; IH-NMR (300 MHz, $CDCl_3$+$CD_3OD$), δ=7.30-7.80 (m, 13 H, Ar-H), 4.99, 5.05 (2d, 2 H, J=12.5 Hz, $CH_2Ph$), 4.34-4.45 (m, 2 H), 4.24-4.32 (m, 3 H), 4.08-4.20 (m, 2 H), 3.78-3.85 (m, 2 H), 3.75 (d, 2 H, 7.0 Hz, $CH_2$), 3.53-3.62 (m, 1 H), 1.28 (d, 3 H, J=7.0 Hz, $CH_3$), 1.06 (d, 3 H, J=6.0 Hz, $CH_3$) . ES-MS; Calc. for $C_{44}H_{37}O_9N_4$; 645.67. Found; 646.0 (M+H), 669.3 (M+Na).

Example 2: Synthesis of donors for first level library

Synthesis of donors for the first level of the library is summarized in Reaction Scheme II.

Preparation of compound 9

A mixture of N-acetyl-D-galactosamine, 20 g, 90.4 mmol, 20 mL of benzaldehyde dimethyl acetal and 200 mg of p-toluenesulfonic acid in 500 mL of dry acetonitrile was stirred at 60°C for 5 hours. After cooling to room temperature, the solid was filtered, washed with $CH_2Cl_2$ (3×20 mL) and dried in vacuo to give compound 9, a solid, 22 g, 88%. [α]D +133.0 (c1.0, $H_2O$). $^1$H-NMR (DMSO-d6) : δ=7.35-7.71 (m, Ar-H), 5.62 (s, 1 H, CHPH), 5.12 (d, 1 H, J=3.0 Hz, H-1), 4.21 (bd, 1H, J=3.5 Hz, H-4), 4.12 (dd, 1 H, J=3.5, I 1.0 Hz, H-2), 4.06 (m, 2 H, H-6), 3.92 (m, 1 H, H-3), 3.85 (m, I H, H-5), 1.90 (s, 3 H, NAc). $^{13}$C-NMR: δ=99.7 (CHPh), 91.4 (C-1).

**Preparation of compound 1**0

To a solution of compound **9**, 20 g, 64.7 mmol in 150 mL of dry pyridine was dropped 8.9 mL of benzoyl chloride in 40 mL of $CH_2C_{12}$ at -25°C under argon in 30 min. and stirred for 2 hours. After adding 5 mL of ethanol the solvent was removed *in vacuo*. Aqueous workup ($CH_2Cl_2$) and recrystallization from ethyl acetate gave compound 10, a solid, 16 g, 62%. [α]D +182.0 (cl, MeOH) ; $^1$H-NMR: 87.30-8.10 (m, Ar-H), 6.05 (s, 1 H, NH), 4.00-5.40 (m), 1.88 (s, 3 H, NAc).

**Preparation of compound 11**

To a solution of compound **10**, 10 g, 24.9 mmol in 100 mL of dry $CH_2Cl_2$ was added 7 mL of trichloroacetonitrile and 0.5 mL of DBU. The solution was stirred for 2 hours at room temperature and solvent was removed in vacuo. The residue was purified by silica gel column using hexane/ethyl acetate(1:1) to give compound 11, a white powder after freeze drying from benzene, 11.5 g, 85%. [α]D +151.0 (cl.0, $CHCl_3$). IH NMR: δ=8.85 (s, 1 H, NH), 7.408.10 (m, Ar-H), 6.75 (d, 1 H, J=3.5 Hz, H-1), 5.65 (d, 1 H, J=9.0 Hz, NH), 5.60 (s, 1 H, CHPH). 5.50 (dd, 1 H, J=3.5 Hz, H-3), 4.00-5.15 (in), 1.90 (s, 3H, NAc).

**Preparation of compound 12**

To a solution of N-acetyl galactosamine, 50 g, 0.226 mol in 100 ml of dry allyl alcohol was dropped 10 N HCl in 50 ml of THF. After stirring at 50°C overnight the solvent was removed in vacuo and the residue was purified by recrystallization from ethanol to give compound **12**, a solid, 35 g, 59% - [α]D +165.00 (c1.0, $H_2O$). $^1$H NMR (D20): δ=6.00 (m, 1 H, CH=), 5.30 (m, 2 H, =$CH_2$), 4.95 (d, 1 H, J=3.5 Hz, H-1), 3.70-4.30 (m), 2.01 (s, 3 H, NAc).

**Preparation of compound 13**

A mixture of compound **12**, 35 g, 0. 134 mol, 62 mL of benzaldehyde dimethyl acetal and 350 mg of p-toluenesulfonic acid in 600 mL of dry acetonitrile was stirred at 60°C for 4 hours. The solvent was removed in vacuo and the residue was purified by recrystallization from ethanol to give compound 13, a solid, 29 g, 62%. [α]D +155.0 (cl.0, MeOH). $^1$H NMR: 8=7.30-7.60 (m, Ar-H), 5.80 (m, 1 H, CH=), 5.57 (s, 1 H, CHPh), 5.26 (m, 2 H =$CH_2$) 5.00 (d, 1 H, J=3.5 Hz, H-1), 3.70-4.55 (m), 2.90 (d, 1 H, J=10.0 Hz, OH), 2.04 (s, 3 H, NAc).

**Preparation of compound 14**

A solution of 13, 20 g, 57.3 mmol, 40 g of tetra-0-acetyl-bromo-(α-D-galactopyranoside and $Hg(CN)_2$, 24 g in 50 mL of dry benzene/ 50 mL of dry nitromethane were stirred at 50°C overnight under argon. Tetra-0-acetyl-bromo-(α-D-galactopyranoside (25 g) and $Hg(CN)_2$ (15 g) were added and the stirring was continued for 4 hours. The solvent was removed in vacuo and the residue was dissolved in 1000 mL of $CH_2C_{12}$, washed with sat'd $Na_2CO_3$, 30 % KBr and water before drying over $Na_2SO_4$. The solvent was removed in vacuo and the residue was purified by silica gel column (1:1 to 1:4 hexane/ethyl acetate) to give compound **14**, a white powder after freeze drying from dioxane, 25 g, 64%. [α]D +96.00 (c2.0, MeOH). $^1$H-NMR (300 MHz, $CDCl_3$) : 8=7.40-7.70 (m, Ar-H), 5.88 (m, 1 H, CH=), 5.65 (d, 1 H, J=9.5 Hz, NH), 5.55 (s, 1 H, CHPH), 5.35 (m, 1 H, H-4b), 5.25 (m, 3 H), 5.03 (d, I H, J=3.5 Hz, H-1a), 4.96 (dd, 1 H, J=3.5, 10.0 Hz, H-3b), 4.75 (d, 1 H, J=7.5 Hz, H-1b). 3.6-4.70 (m), 2.14, 2.04, 1.98, 1.96 (4 s, 15 H, 5 Ac).

**Preparation of compound 15**

To a solution of compound **14**, 23 g, 33 mmol and [bis(methyldiphenylpilosphine)1(1,5-cyclooctadiene)-iridium (1) hexafluoroptiosphate, 500 mg in 500 mL of dry THF was bubbled argon for 15 min. and hydrogen for 5 min. The solution was stirred at room temperature for 4 hours. 15 g of $I_2$, 800 mg of dimethylaminopyridine, 10 mL of pyridine and 80 mL of water were added and the solution was stirred at room temperature overnight. Sodium sulfate (5%, 200 mL) was added and THF was removed *in vacuo*. Aqueous workup ($CH_2Cl_2$) and silica gel purification (5:10:2 hexane/ethyl acetate/methanol) gave compound **15**, a white powder after freeze drying from dioxane, 12.5 g, 58%. [α]D+55.6° (cl.0, MeOH) . $^1$H-NMR(300 MHz, $CDCl_3$) : δ=7.35-7.60 (m, Ar-H), 6.50 (d, 1 H, J=9.0 Hz, NH), 5.45 (s, 1 H, CHPh), 5.33 (d, 1 H, J=3.5 Hz, H-1a), 5.15 (m, 2 H, H-2b & H-4b), 4.96 (2d, 1 H, J=3.5, 10.5 Hz, H3b), 4.68 (d, 1 H, J=8.0 Hz, H-1b), 3.60-4.50 (m), 2.11, 2.02, 2.00, 1.96, 1.94 (5 s, 15 H, 5 Ac).

**Preparation of compound 16**

To a solution of compound **15**, 7.5 g, 11.5 mmol in 100 mL of dry $CH_2Cl_2$ was added 2.6 mL of trichloroacetonitrile

and 0.25 mL of DBU. The solution was stirred for 2 hours at room temperature and solvent was removed in vacuo. The residue was purified by silica gel column using hexane/ethyl acetate(1:2) to give compound **16**, a white powder after freeze drying from benzene, 5.6 g, 61%. [α]D +81.2 (cl.0, $CH_2Cl_2$). $^1$H-NMR (300 MHz, $CDCl_3$): δ=8.75 (s, 1 H, NH), 7.35-7.60 (m, Ar-H), 6.62 (d, 1H, J=3.5 Hz, H-1a), 5.80 (d, I H, J=8.0 Hz, NH), 5.52 (s, 1 H, CHPh), 5.40 (2d, 1 H, J=1.0, 3.5 Hz, H-4b), 5.25 (dd, 1 H, J=8.0, 10.0 Hz, H-2b), 5.01 (dd, 1 H, J=3.5, 10.5 Hz, H-2a), 4.90 (d, 1 H, J=8.0 Hz, H-1b), 3.80-4.80 (m), 2.15, 2.04, 2.02, 2.00, 1.96 (5 s, 15 H, 5 Ac).

Example 3: Synthesis of donors for second level library

Synthesis of donors for the second level of the library is summarized in Reaction Scheme III.

**Preparation of compound 17**

K. Fufase, *et al., Tetrahedron Lett.*, **36**: 7455-7458. To a solution of D-glucosamine hydrochloride, 34 g, in 1 L of water was added trichloroethyl chloroformate, 33 mL dropwise over 2-3 hours at 0°C. After stirring at room temperature overnight, the solid was filtered out and washed with water and then ether. The solid was recrystallized from ethanol to give N-troc-D-glucosamine, 59.5 g, 98%. A solution of N-troc-D-glucosamine, 13.5 g, 38.2 mmol in 100 mL of allyl alcohol with 5% HCl was stirred at 100°C for 30 min. After cooling to room temperature, allyl alcohol was remove in vacuo and the residue was dissolved in 250 mL of acetonitrile. 12 ml of $PhCH(OMe)_2$ and 100 mg of TSOH were added. The mixture was stirred at room temperature over night under argon. 10 g of $Na_2CO_3$ was added and the mixture was stirred for 10 min. Solid was removed and washed with acetone (5x5mL). The solvent was removed and the solid was purified by crystallized from ethanol to give compound 17, a solid, 12 g, 65%. $^1$H-NMR (300 MHz, $CDCl_3$). δ=7.30-7.60 (m, 5 H, Ar-H), 5.80-6.00 (m, 1 H, CH=), 5.55 (s, 1 H, CHPh), 5.20-5.40 (m, 2 H, $=CH_2$), 4.92 (d, 1H, J=3.0 Hz, H-1), 4. 82, 4.69 (2 d, 2 H, J=12.0 Hz, $CH_2$), 3.70-4.30 (m), 3.59 (t, 1 H, J=8.0. Hz, H-4), 2.67 (d, 1H, J=2.0 Hz, OH).

**Preparation of compound 18**

A solution of compound **17**, 8 g, 16.6 mmol in 20 mL of pyridine and 10 mL of acetic anhydride was stirred at room temperature overnight. The solvent was removed and the residue was purified by crystallization from ethanol to give compound **18**, a solid, 4.7 g, 54%. [α]D +58.5 (cl, ethyl acetate); $^1$H-NMR (300 MHz, $CDCl_3$) : δ=7.30-7.50 (m, 5 H, Ar-H), 5.806.00 (m, 1 H, CH=), 5.53 (s, 1 H, CHPH), 5.38 (t, 1 H, J=10 Hz, H-3), 5.20-5.35 (m, 2 H, $=CH_2$), 4.93 (d, 1 H, J=3.5 Hz, H-1), 4.79, 4.68 (2 d, 2 H, J=12.0 Hz, $CH_2$), 3.65-4.35 (m), 2. 1 0 (s, 3 H, $CH_3$)

**Preparation of compound 19**

To a solution of compound **18**, 9.1 g, 17.4 mmol in 300 mL of THF was added [bis(methyldiphenylphosphine)] (1,5-cyclooctadiene)-iridium(1) hexafluorophosphate, 350 mg. Argon was passed to the solution for 10 min. following by hydrogen, 20 min. The solution was stopped and stirred at room temperature for 4 hours. 500 mg of DMAP, 6.25 mL of pyridine, 50 mL of water and 9.35 g of $I_2$ were added. The mixture was stirred at room temperature overnight. THF was removed and the mixture was dissolved in 1 L of $CH_2Cl_2$. The solution was washed with sat. sodium carbonate, 1N HCl and water, dried over $Na_2SO_4$. The solvent was removed and the residue was purified by silica gel column using hexane/ethylacetate=7:3 to give compound **19**, a white powder after freeze drying from dioxane, 5.5 g, 65%. [α]D +60.0 (cl, ethyl acetate); $^1$H-NMR (300 MHz, $CDCl_3$): δ=7.30-7.50 (m, 5 H, Ar-H), 5.53 (s, 1H, CHPh), 5.52 (d, 1 H, J=10.0 Hz, NH), 5.43 (t, 1 H, J=10 Hz, H-3), 5.33(d, 1H, J=3.5 Hz, H-1), 4.81, 4.66 (2 d, 2 H, J=12.0 Hz, $CH_2$), 3.65-4.35 (m), 3.17 (dd, 1H, J=1.0, 3.5 Hz, OH), 2.06 (s, 3 H, $CH_3$).

**Preparation of compound 20**

A solution of compound **19**, 5.5 g, 11 mmol, 2.42 mL of tricholoroacetonitrile and 6 drops of DBU in 70 mL of $CH_2Cl_2$ was stirred at room temperature for 2 hours under argon. The solvent was removed *in vacuo* and the residue was purified by silica gel column using hexane/ethyl acetate(7:3) to give compound **20**, a white powder after freeze drying from benzene, 5.59 g, 81%. [α]D +59.0 (cl, ethyl acetate); $^1$H-NMR (300 MHz, $CDCl_3$): 8=8.80 (s, 1 H, NH), 7.307.50 (m, 5 H, Ar-H), 6.40 (d, 1H, J=3.5 Hz, H-1), 5.56 (s, 1H, CHPh), 5.46 (t, 1 H, J=10 Hz, H-3), 5.36 (d, 1 H, J=9.0 Hz, NH), 4.71 (dd, 2 H, J=12.0 Hz, $CH_2$), 4.35 (dd, 1 H, J=5.0, 10.0 Hz, H-4), 4.27 (m, 1 H, H-2), 3.75-4.00 (m), 2.11 (s, 3 H, $CH_3$).

**Preparation of compound 21**

G. Grundler and R. R. Schmidt, *Libigs Ann. chem.,* 1984, 1826-1847.
To a solution of 2.0 mL of HClO4 (70%) in 300 mL of acetic anhydride was added lactose, 100 g, 0.29 mol by portion to keep the temperature between 30 to 35°C. After adding of 15 g of red phosphorous, the mixture was cooled in ice-salt bath and 90 g (29 mL) of $Br_2$ was dropped to keep the temperature below 20°C. 15 mL of water was added in 15 min. The solid was filtered out and washed with acetic acid. The mixture was added to a solution of 100 g of zinc and 11 g of $CuSO_4$ in 290 mL of water and 200 mL of acetic acid in 2 hour at -20°C. The mixture was stirred at -20°C for 2 hours and the solid was filtered out and washed with acetic acid. The solvent was removed and the residue was purified by silica gel column using hexane/ethyl acetate (1:1) to give compound **21**, a white powder after freeze drying from benzene, 100 g, 62%. $^1$H-NMR (300 MHz, $CDCl_3$) : 8=6.32 (dd, 1 H, J=2.0, 6.5 Hz, =CH), 5.32 (m, 1 H, =CH), 5.27 (dd, I H, J= 1.0, 3.5 Hz, H-4'), 5.09 (dd, 1 H, J=8.0, 11.0 Hz, H-2'), 4.92 (dd, 1H, J=3.5, 10.5 Hz, H-3'), 4.74 (dd, 1H, J=3.5, 6.0 Hz, H-3), 4.60 (d, I H, J=8.0 Hz, HI'), 3.80-4.40 (m), 2.07, 2.03, 2.00, 1.97, 1.96, 1.89 (6 s, 18 H, 6 Ac).

**Preparation of compound 22**

G. Grundler and R. R. Schmidt, *Libigs Ann. Chem.,* 1984, 1826-1847.
To a solution of compound **21**, 100 g, 0.179 mol and 300 g of ceric ammonium nitrite (CAN) was added 20 g of sodium azide at -20°C under nitrogen. After stirring at -20°C for 6 hours, 1 L of water was added and the mixture was extracted with ether (5x500 mL). Ether was washed with water and evaporated. The residue was dissolved in 1 L of dioxane and 100 g of $Na_2NO_2$ in 30 mL of water was added. the mixture was stirred at 80°C for 6 hours. 500 mL of water was added and the mixture was extracted with $CH_2Cl_2$ (5x400 mL). The solution was washed with water and dried over $Na_2SO_4$. The solvent was removed in vacuo and the residue was purified by silica gel column using hexane/ethyl acetate (1:1 to 1:2) as eluant to give compound 22, a whiter powder after freeze drying from benzene, as a mixture of α, β-isomers.

**Preparation of compound 23**

G. Grundler and R. R. Schmidt, *Libigs Ann. Chem.,* 1984, 1826-1847.
To a solution of compound **22**, 20 g, 32.4 mmol in 300 mL of $CH_2Cl_2$ was added 8 mL of $Cl_3CCN$ and 1.7 mL of DBU. The mixture was stirred at room temperature for 3 hours under argon. The solvent was removed *in vacuo* and the residue was purified by silica gel column using hexane/ethyl acetate from 1:1 to 2:3 to give compound **23**, a white powder after freeze drying from benzene, 11.5 g, 47%. $^1$H-NMR (300 MHz, $CDCl_3$): δ=8.80 (s, 1 H, NH), 6.44(d, 1 H, J=3.5 Hz, H-1), 5.51 (dd, 1 H, J=9.0, 10.5 Hz, H-3), 5.36 (dd, 1 H, J=1.0, 3.0 Hz, H-4'), 5.13 (dd, 1 H, J=8.0, 10.0 Hz, H-2'), 4.96 (dd, 1 H, J=3.5, 10.5 Hz, H-3'), 4.51 (d, I H, J=7.5 Hz, H-1'), 3.70-4.50 (m), 3.62 (dd, 1 H, J=3.5, 10.5 Hz, H-2), 1.90-2.20(6 s, 18 H, 6 Ac).

**Preparation of compound 24**

T. J. Martin and R. R. Schmidt, *Tetrahedron Lett.*, **36**: 7455-7458.
A solution of sialic acid, 10 g and IR120+ resin, 4 g in 1000 mL of dry methanol was stirred at room temperature overnight under argon. The resin was filtered out and washed with methanol. The solvent was removed to give compound **24**, a solid, 10.65 g, 100%.

**Preparation of compound 25**

T. J. Martin and R. R. Schmidt, *Tetrahedron Lett.*, **36**: 7455-7458.
To a solution of 20 mL of acetic anhydride was added 0.1 mL of $HClO_4$. Compound **24**, 10.65 g, was added to the mixture by portion to keep the temperature between 30 to 35°C. After finishing adding the mixture was stirred at room temperature for 3 hours. The solution was poured onto ice water and kept at room temperature overnight. The solution was extracted with chloroform (3x100 mL) and washed with sat. $NaHCO_3$ and water, dried over $Na_2SO_4$. The solvent was removed *in vacuo* and the residue was purified by silica gel column using hexane/ethyl acetate/methanol (5:20:1) to give compound 25, a white powder after freeze drying from dioxane, 6 g, 76% as a mixture of α,p-isomers $^1$H-NMR of major isomer (300 MHz, $CDCl_3$) : 6=6.15 (d, 1 H, J=10.0 Hz, NH), 5.39 (dd, 1 H, J=2.0, 4.5 Hz, H-7), 5.23 (m, 1 H, H-8), 5.16 (t, 1H, J=9.0 Hz, H-4), 4.97 (s, 1 H, OH), 4.60 (dd, 1 H, J=2.0, 10.0 Hz, H-9a), 4.18 (t, 1 H, J=10.0 Hz, H-5), 4.03 (dd, 1 H, J=8.0, 11.5 Hz, H-9b), 3.85 (s, 3 H, $CH_3$), 2.22 (d, 1H, J=7.0 Hz, H-3e), 2.15, 2.07, 2.02, 2.01, 1.90, (5 s, 15 H, 5 Ac). 2.02 (m, 1 H, H-3a).

**Preparation of compound 26**

T J. Martin and R. R. Schmidt, *Tetrahedron Lett.*, **36**: 7455-7458.
To a solution of compound **25**, 7.2 g, 14.4 mmol in 150 mL of dry acetonitrile was added 6 mL of EtNi-$Pr_2$ and 4.5 mL of phospholoride diethyl ester. The solution was stirred at room temperature for 10 min. and the solvent was removed *in vacuo.* The residue was purified by silica gel column using hexane/acetone 1:1 to give compound 26, a syrup, 8.5 g, 97% as a mixture of ($\alpha$,$\beta$-isomers. $^1$H-NMR (300 MHz, $CDCl_3$) : $\delta$=2.80 (dd, 0.33 H, H-2e$\beta$). 2.50 (dd, 0.67H, H-2e $\alpha$).

Example 4: Generation of first level library

GSTA is glycosylated separately with the protected Tn and TF donors to produce first level libraries with four glycoforms each:

1. Tn: 00, Tn0, 0Tn, TnTn
2. TF: 00, TF0, 0TF, TFTF

where "0" means no glycosylation.
GSTA also is reacted with Tn and TF as a mixture. The result is a first level library that contains nine glycoforms:

3. Tn + TF: 00, Tn0, 0Tn, TnTn, TF0, 0TF, TFTF, TnTF, TFTn

As can be seen, the first level libraries 1, 2 and 3 above are different in composition and/or size. Library 3 formed with mixed Tn and TF glycosyl donors contains all the glycoforms that occur in libraries 1 and 2, formed when Tn and TF are used separately as glycosyl donors, in addition to other components that arise from the use of mixed donors. A library formed with a mixture of two donors is therefore more comprehensive than a library formed by combining two libraries produced when the two donors are reacted with the core peptide separately. Generation of a first level library is summarized in Reaction Scheme IV.

**Preparation of compounds 27**

A solution of compound **8**, 0.6 g, 0.925 mmol, compound 11, 2 g, 3.73 mmol, compound 16, 2.45 g, 3.088 mmol and 3.5 g of 3 A molecular sieves in 20 mL of dry THF was stirred at room temperature for 10 min. under argon and cooled to -20°C. Ten mL of 0.1 mol of $BF_3EtO_2$ in dry THF was added in 5 min. After stirring at -20°C for 1 hour, the reaction mixture was then warmed to room temperature. The molecular sieves were filtered out and washed with ethyl acetate (3x20 mL). The solvent was removed *in vacuo* and the residue was purified by silica gel column using hexane/ethyl acetate/methanol (10:10:1.5) to give 2.3 g of a mixture as a white powder after freeze drying from dioxane.

**Preparation of compounds 28**

A solution of compound **27**, 2.3 g in 40 mL of dry pyridine and 5 mL of dry acetic anhydride was stirred at room temperature overnight under argon. The solvent was removed in vacuo and the residue was purified by silica gel column using hexane/ethyl acetate/methanol (10:10:1.5) to give 2.34 g of a white powder after freeze drying from dioxane.

**Preparation of compounds 29**

A solution of compound **28**, 1.9 g in 30 mL of 80% aqueous acetic acid was stirred at 80°C for 2 hours. The solvent was removed *in vacuo* and the residue was purified by silica gel column using hexane/ethyl acetate/methanol (5:10:3) to give 1.61 g of a white powder after freeze drying from dioxane.

**Preparation of compound 30**

A mixture of compound **29**, 150 mg and 5 mL of 0.1 N NaOH in 5 mL of methanol was stirred at room temperature for 24 hours. Then 5 mL of 0.1 N NaOH and 5 mL of methanol was added and the stirring was continued for 24 hours. IR120+ resin was added to adjust pH=4.5. The resin was filtered out and washed with water (5x5 mL). The water was removed in *vacuo* and the residue was purified by P-2 column using water as eluant to give a solid, 13.1 mg.

ES-MS

30-1: Calc. for $C_{20}H_{35}O_{12}N_5$, 537.50. Found, 538.55 (M+H)
30-2: Calc. for $C_{28}H_{48}O_{17}N_6$, 740.31. Found, 741.41 (M+H)
30-3: Calc. for $C_{26}H_{45}O_{17}N_5$, 699.64. Found, 700.43 (M+H)
30-4: Calc. for $C_{40}H_{68}O_{27}N_6$, 1064.41. Found, 1065.67 (M+H)
30-5: Calc. for $C_{34}H_{58}O_{22}N_6$, 902.36. Found, 903.51 (M+H)
30-6: Calc. for $C_{34}H_{58}O_{22}N_6$, 902.36. Found, 903.51 (M+H)

Example 5: Generation of second level libraries

Unglycosylated serine and threonine residues in the first level libraries are temporarily blocked by acetylation to prevent the next set of glycosyl donors from reacting with unreacted sites. Following purification the 4,6-benzylidenyl protecting groups of all glycoforms are selectively removed by acid cleavage to create additional glycosylation sites on all the existing carbohydrate structures. GlcNAc, sialic acid (S) and N-acetyllactosamine (L) are reacted with the existing carbohydrate structures of the glycopeptides of each first level library, to produce second level libraries with new structures. Sialic acid and N-acetyllactosamine react at the 6-O- position of N-acetylgalactosamine common to the glycosylated Tn and TF components of the first level libraries.

For example, the following second level libraries are created when libraries 1, 2 and 3 of Example 1 are reacted with sialic acid:

4. 1 + S: 00, Tn0, STn0, 0Tn, 0STn, TnTn, TnSTn, STnTn, STnSTn
5. 2 + S: 00, TF0, STF0, 0TF, 0STF, TFTF, TFSTF, STFTF, STFSTF
6. 3 + S: 00, Tn0, STn0, 0Tn, 0STn, TnTn, STnTn, TnSTn, STnSTn, TF0, STF0, 0TF, OSTF, TFTF, STFTF, TFSTF, STFSTF, TnTF, STnTF, TnSTF, STnSTF, TFTn, STFTn, TFSTn, STFSTn

Second level library 6 is more comprehensive than 4 and 5 combined.

The number of components may be increased further by using mixed donors, as in library 7. When library 3 from the first level is reacted with a mixture of sialic acid and N-acetyllactosamine, the following library is produced:

7. 3 + SL: 00, Tn0, STn0, 0Tn, 0STn, TnTn, STnTn, TnSTn, STnSTn, TF0, STF0, 0TF, OSTF, TFTF, STFTF, TFSTF, STFSTF, TnTF, STnTF, TnSTF, STnSTF , TFTn, STFTn, TFSTn, STFSTn, LTn0, 0LTn, LTnTn, TnLTn, LTnLTn, LTF0, OLTF, TFLTF, LTFTF, LTFLTF, STnLTn, LTnSTn, LTFSTF, STFLTF

Additional second level libraries can be formed by mixing each of libraries 1, 2 and 3 with N-acetyllactosamine, and by mixing each of libraries 1 and 2 with a mixture of sialic acid and N-acetyllactosamine.

The ultimate size and definition of the library is controlled by the number and identity of donors, pre-blocking of defined sites on the peptide, and use of split-, mix-split type of synthesis, as described in Plunkett et al., Scientific American, April 1997, 68-73, the contents of which are incorporated herein by reference. Generation of a second level libraries is summarized in Reaction Schemes V, VI, VII and VII.

**Synthesis of second level library with GlcNAc as donor**

**Preparation of compound 31**

A solution of compounds **29**, 0.4 g, compound 20, 1.2 g and 2 g of 3 A molecular sieves in 15 mL of dry acetonitrile was stirred at room temperature for 10 min. under argon. The mixture was cooled to -20°C and 3 mL of 0. 1 mol of TMS-OTF in dry acetonitrile was added in 5 min. The solution was stirred at -20°C for 1 hour and warmed to room temperature. The molecular sieves were filtered out and washed with ethyl acetate (5x5 mL). The solvent was removed in vacuo and the residue was purified by silica gel column using hexane/ethyl acetate/methanol (5:10:2) to give 0.64 g of a white powder after freeze drying from, dioxane.

**Preparation of compound 32**

A solution of compound **31**, 0.63 g and 4 g of activated zinc in 50 mL of 80% acetic acid in ethyl acetate was stirred at room temperature for 2 hours. Zinc was filtered out and washed with ethyl acetate (5x20 mL). The solvent was removed *in vacuo* and the residue was dissolved in 15 mL of dry pyridine and 5 mL of dry acetic anhydride. The solution was stirred at room temperature for 12 hours. The solvent was removed *in vacuo* and the residue was purified by silica gel column using hexane/ethyl acetate/methanol (5:10:2) to give 0.60 g of a white powder after freeze drying from dioxane. A solution of above solid, 200 mg, in 200 mL of 80% of aqueous acetic acid was stirred at 80°C for 2 hours and the solvent was removed in *vacuo.* The residue was dissolved in 5 mL of methanol and 20 mL of 0.1 N NAOH was added and the mixture was stirred at room temperature for 24 hours. IR120+ resin was added to adjust pH=4.5 and the resin was filtered out and washed with water (5x5 mL). Water was removed in *vacuo* and the residue was purified by P-2 column to give a solid, 15 mg.

ES-MS

| | |
|---|---|
| 32-1: | Calc. for $C_{28}H_{48}0_{17}N_6$, 740.31. Found, 741.30 (M+H) + |
| 32-2: | Calc. for $C_{36}H_{61}O_7$, 943.39. Found, 944.46 (M+H) + |
| 32-3: | Calc. for $C_{36}H_{61}O_{22}N_7$, 943.39. Found, 944.46 (M+H) + |
| 32-4: | Calc. for $C_{44}H_{74}O_{27}N_8$, 1146.46. Found, 574.50 (M+2H) 2+ |
| 32-5: | Calc. for $C_{34}H_{58}O_{22}N_6$, 902.36. Found, 903.41 (M+H) + |
| 32-6: | Calc. for $C_{42}H_{71}O_{27}N_7$, 1105.44. Found, 1106.72 (M+H) + |
| 32-7: | Calc. for $C_{42}H_{71}O_{27}N_7$, 1105.44. Found, 1106.72 (M+H) + |
| 32-8: | Calc. for $C_{56}H_{94}O_{37}N_8$, 1470.57. Found, 737.00 (M+2H) + |
| 32-9: | Calc. for $C_{50}H_{84}O_{32}N_8$, 1308.52. Found, 1309.57 (M+H)+ |
| 32-10: | Calc. for $C_{50}H_{84}O_{32}N_8$, 1308.52. Found, 1309.57 (M+H)+ |
| 32-11: | Calc. for $C_{42}H_{71}O_{27}N_7$, 1105.44. Found, 1106.72 (M+H)+ |
| 32-12: | Calc. for $C_{42}H_{71}O_{27}N_7$, 1105.44. Found, 1106.72 (M+H)+ |
| 32-13: | Calc. for $C_{48}H_{81}O_{32}N_7$, 1268.16. Found, 635.0 (M+2H) 2+ |
| 32-14: | Calc. for $C_{48}H_{81}O_{32}N_7$, 1268.16. Found, 635.0 (M+2H)2+ |

**Synthesis of second level library with N-acetyl lactosamine as donor**

**Preparation of compound 33**

A solution of compound **29**, 0.4 g, compound **23**, 1.5 g and 2 g of 3A molecular sieves in 15 mL of dry $CH_2Cl_2$ was stirred at room temperature for 10 min. under argon. Three mL of 0.1 mol of $BF_3OEt_2$ in dry $CH_2Cl_2$ was added in 10 min. The solution was stirred at room temperature for 1 hour. The molecular sieves were filtered out and washed with ethyl acetate (5x10 mL) and the solvent was removed *in vacuo*. The residue was purified by silica gel column using hexane/ethyl acetate/methanol (5:10:2) to give 0.43 g of a white powder after freeze drying from dioxane.

**Preparation of compound 34**

A solution of compound **33**, 0.42 g in 30 mL of pyridine and 2 mL of water was passed $H_2S$ gas for 30 min. The solution was stopped and stirred at room temperature for 48 hours. The solvent was removed *in vacuo* and the residue was dissolved in 15 mL of dry pyridine and 5 mL of dry acetic anhydride. The mixture was stirred at room temperature for 12 hours. The solvent was removed in vacuo and the residue was purified by silica gel column using hexane/ethyl acetate/methanol (5:10:2) to give 0.44 g of a white powder after freeze drying from dioxane. A solution of the above solid, 200 mg, was dissolved in 5 mL of methanol and 20 mL of 0.1 N NaOH and the mixture was stirred at room temperature for 24 hours. IR120+ resin was added to adjust pH=4.5 and the resin was filtered out and washed with water (5x5 mL). Water was removed in vacuo and the residue was purified by P-2 column to give a solid, 12.7 mg.

ES-MS

34-1: Calc. for $C_{34}H_{58}O_{22}N_6$, 902.36. Found, 903.46 (M+H)+
34-2: Calc. for $C_{41}H_{71}O_{27}N_7$, 1105.44. Found, 1106.54 (M+H)+
34-3 : Calc. for $C_{42}H_{71}O_{21}N_7$, 1105.44. Found, 1106.54 (M+H) +
34-4: Calc. for $C_{56}H_{94}O_{37}N_8$, 1470.57. Found, 736.60 (M+2H) +
34-5 : Calc. for $C_{40}H_{68}O_{27}N_6$, 1064.41. Found, 1065.54 (M+H) +
34-6: Calc. for $C_{48}H_{81}O_{32}N_7$, 1267 . 49 . Found, 1268.52 (M+H) +
34-7: Calc. for $C_{48}H_{81}O_{32}N_7$, 1267.49. Found, 1268.52 (M+H) +
34-8: Calc. for $C_{68}H_{114}O_{47}N_8$, 1794.68. Found, 898.70 (M+2H) 2+
34-9: Calc. for $C_{62}H_{104}O_{42}N_8$, 1632.62. Found, 817.64 (M+2H) +
34-10: Calc. for $C_{62}H_{104}O_{42}N_8$, 1632.62. Found, 817.64 (M+2H) +
34-11: Calc. for $C_{48}H_{81}O_{32}N_7$, 1267.49. Found, 1268 . 52 (M+H)+
34-12: Calc. for $C_{48}H_{81}O_{32}N_7$, 1267.49. Found, 1268. 52 (M+H) +
34-13: Calc. for $C_{54}H_{91}O_{37}N_7$, 1430.30. Found, 1430.71 (M+H)+
34-14: Calc. for $C_{54}H_{91}O_{37}N_7$, 1430.30. Found, 1430.71 (M+H ) +

**Synthesis of second level library with sialic acid as donor**

**Preparation of compound 35**

A solution of compounds **29**, 0.4 g, compound 26, 0.8 g and 2 g of 3A molecular sieves in 15 mL of dry THF was stirred at room temperature for 10 min. under argon and cooled to -20°C. Two mL of 0.1 mol of TMS-OTF in dry THF was added in 5 min. The solution was stirred at -20°C for 1 hour. The molecular sieves was filtered out and washed with ethyl acetate (5x10 mL) and the solvent was removed *in vacuo*. The residue was purified by silica gel column using hexane/ethyl acetate/methanol (5:10:3) to give 0.55 g of a white powder after freeze drying from dioxane.

**Preparation of compound 36**

A solution of compound **35**, 0.2 g in 10 mL of 0.1 N NaOH and 5 mL of methanol was stirred at room temperature for 24 hours. IR120+ resin was added to adjust pH=4.5 and the resin was filtered out and washed with water (5x5 mL). Water was removed in vacuo and the residue was purified by P-2 column to give a solid, 18.3 mg.

ES-MS

36-1: Calc. for $C_{31}H_{52}O_{20}N_6$, 828.32. Found, 829.60 (M+H)+
36-2: Calc. for $C_{39}H_{65}O_{25}N_7$, 1031.40. Found, 1032.80 (M+H)+
36-3: Calc. for $C_{39}H_{65}O_{2}5N_7$, 1031.40. Found, 1032.80 (M+H) +
36-4: Calc. for $C_{50}H_{82}O_{33}N_8$, 1322.50. Found, 662.00 (M+2H)2+
36-5: Calc. for $C_{37}H_{62}O_{25}N_6$, 990.37. Found, 991.70 (M+H) +
36-6: Calc. for $C_{45}H_{75}O_{30}N_7$, 1193 .45. Found, 1194. 80 (M+H)+
36-7: Calc. for $C_{45}H_{75}O_{30}N_7$, 1193.45. Found, 1194.80 (M+H)+
36-8: Calc. for $C_{62}H_{102}O_{43}N_8$, 1646.60. Found, 824.20 (M+2H) 2+
36-9: Calc. for $C_{56}H_{92}O_{38}N_8$, 1484.55. Found, 743.60 (M+2H)2+
36-10: Calc. for $C_{56}H_{92}O_{38}N_8$, 1484.55. Found, 743.60 (M+2H) 2+
36-11: Calc. for $C_{45}H_{75}O_{30}N_7$, 1193.45. Found, 1194.80 (M+H)+
36-12: Calc. for $C_{45}H_{75}O_{30}N_7$, 1193.45. Found, 1194.80 (M+H) +
36-13: Calc. for $C_{51}H_{85}O_{35}N_7$, 1356.22. Found, 1356.60 (M+H)+
36-14: Calc. for $C_{51}H_{85}O_{35}N_7$, 1356.22. Found, 1356.60 (M+H) +

**Synthesis of second level library with GlcNAc and sialic acid as donors**

**Preparation of compound 37**

A solution of compounds **29**, 0.2 g, compound **26**, 0.22 g and compound **20**, 0.22g with 2 g of 3A molecular sieves in 15 mL of dry THF was stirred at room temperature for 10 min. under argon and cooled to -20°C. One mL of 0.1 mol of TMS-OTF in dry THF was added in 5 min. The solution was stirred at -20°C for 1 hour. The molecular sieves was filtered out and washed with ethyl acetate (5x10 mL) and the solvent was removed *in vacuo.* The residue was purified by silica gel column using hexane/ethyl acetate/methanol (5:10:1) to give 0.27 g of a white powder after freeze drying from dioxane.

**Preparation of compound 38**

A solution of compound **37**, 0.27 g and 1 g of activated zinc in 20 mL of 80% acetic acid in ethyl acetate was stirred at room temperature for 2 hours. Zinc was filtered out and washed with ethyl acetate (5x20 mL). The solvent was removed *in vacuo* and the residue was dissolved in 4 mL of dry pyridine and 2 mL of dry acetic anhydride. The solution was stirred at room temperature for 12 hours. The solvent was removed *in vacuo* and the residue was purified by silica gel column using hexane/ethyl acetate/methanol (5:10:1) to give 0.25 g of a white powder after freeze drying from dioxane. A solution of above solid, 0.25 g, in 10 mL of 80% of aqueous acetic acid was stirred at 80°C for 2 hours and the solvent was removed in *vacuo.* The residue was dissolved in 3 mL of methanol and 7 mL of 0.1 N NaOH was added and the mixture was stirred at room temperature for 24 hours. IR120+ resin was added to adjust pH=4.5 and the resin was filtered out and washed with water (5x5 mL). Water was removed *in vacuo* and the residue was purified by P-2 column to give a solid, 16.1 mg.

Example 6: Screening-of GSTA libraries

An ELISA assay with antibodies that bind to a solid phase of either Ovine Submaxillary Mucin (OSM) or CA27.29 were used in an inhibition format to screen the GSTA libraries, with library components being used as inhibitors. Wells were coated with 110 μL of antigen diluted in PBS and allowed to stand overnight at 4°C. One μg/mL OSM or 10 U/mL CA27.29 were used in the assay. On the day of the assay, the wells were aspirated and washed twice with PBS, then 200 μL of 2% BSA blocking solution was added to each well. The wells were incubated 2 hours at room temperature.

The monoclonal antibodies and inhibitors were diluted in 1% FBS/PBS to various concentrations using glass culture tubes. Monoclonal antibody and inhibitor were combined in pre-incubation tubes. The zero inhibition tube received monoclonal antibody and 1% FBS/PBS diluent. The preincubation for each plate was completed exactly one hour prior to the end of the blocking incubation. When the blocking incubation was complete, the wells were aspirated and washed 4 times with TPBS (PBS + 0.05% Tween 20), and 100 μL of the pre-incubation mixture was added to duplicate wells. Blank and substrate negative control wells received 100 μL of 1% FBS/PBS diluent only.

Wells were incubated for 90 minutes at room temperature. Just prior to the end of incubation, goat anti-mouse IgG, H+L, HRP labelled was diluted with 1% FBS/PBS. The wells were aspirated and washed 4 times with TPBS, and 90 μL of diluted goat anti-mouse HRP was added to all wells except the substrate negative control wells, which received 90 μL of 1% FBS/PBS.

The wells were incubated for 90 minutes at room temperature, and then aspirated and washed 4 times with TPBS. Equal volumes of ABTS and peroxide solution B substrate were mixed, and 100 μL was added to each well. The plate was immediately put into a plate reader and read on kinetic mode at wavelength 405-490 nm, 10 minute read, 20 second interval. Data were expressed in mOD/min, and results are shown in Table 3 and Figure 8:

**Table 3.**
**Inhibition of MAbs with 5 Glycopeptide Libraries June 23, 1997 970623.XLS**

| MAb | | CC49 | | B72.3 | | B195.3R11 | | B239.9R84 | | B27.29 | |
|---|---|---|---|---|---|---|---|---|---|---|---|
| Solid Phase | | OSM | | OSM | | OSM | | OSM | | CA27.29 | |
| | | | | | | | | | | | |
| Inhibitor | Final Conc. | Vmax | % Inhibition | Vmax | % Inhibition | Vmax | % Inhibition | Vmax | % Inhibition | Vmax | % Inhibition |
| | (µg/mL) | (mOD/min.) | | (mOD/min.) | | (mOD/min.) | | (mOD/min.) | | (mOD/min.) | |
| | | | | | | | | | | | |
| No Inhibitor Control | | 121.6 | 0.0 | 127.7 | 0.0 | 130.5 | 0.0 | 113.1 | 0.0 | 120.2 | 0.0 |
| | | | | | | | | | | | |
| # 9, Tn, TF | 60 | 121.6 | 0.0 | 124.9 | 2.2 | 127.0 | 2.7 | 114.7 | -1.4 | 120.7 | -0.4 |
| | 120 | 123.5 | -1.6 | 128.1 | -0.3 | 131.6 | -0.8 | 115.7 | -2.3 | 124.1 | -3.2 |
| | | | | | | | | | | | |
| #10, Tn, TF, STn, STF | 150 | 122.4 | -0.7 | 48.4 | 62.1 | 119.2 | 8.7 | 96.7 | 14.5 | 124.1 | -3.2 |
| | 300 | 124.8 | -2.6 | 19.5 | 84.7 | 100.0 | 23.4 | 70.3 | 37.9 | 121.6 | -1.2 |
| | | | | | | | | | | | |
| #11, Tn, TF, GTn, | 150 | 130.3 | -7.2 | 127.8 | -0.1 | 139.1 | -6.6 | 119.8 | -5.9 | 121.7 | -1.2 |
| | 300 | 118.2 | 2.8 | 126.8 | 0.7 | 131.1 | -0.5 | 112.2 | 0.8 | 117.7 | 2.1 |
| | | | | | | | | | | | |
| #12, TN, TF, STn, STF | 300 | 119.5 | 1.7 | 116.5 | 8.8 | 121.0 | 7.3 | 106.0 | 6.3 | 115.8 | 3.7 |
| GTn, GTF | 600 | 120.7 | 0.7 | 91.7 | 28.2 | 111.3 | 14.7 | 108.3 | 4.2 | 121.0 | -0.7 |
| | | | | | | | | | | | |
| #13, Tn, TF, ? | 150 | 124.6 | -2.5 | 128.6 | -0.7 | 132.3 | -1.4 | 116.9 | -3.4 | 121.9 | -1.4 |
| | 300 | 124.4 | -2.3 | 138.4 | -8.4 | 139.9 | -7.2 | 114.6 | -1.3 | 118.6 | 1.4 |
| | | | | | | | | | | | |
| | | | | | | | | | | | |
| Final Concentrations of Mab's used: | | CC49 | 20ng/mL | B72.3 | 10ng/mL | B195.3R11 | 75/ng/mL | B239.9R84 | 25ng/mL | B27.29 | 20ng/mL |

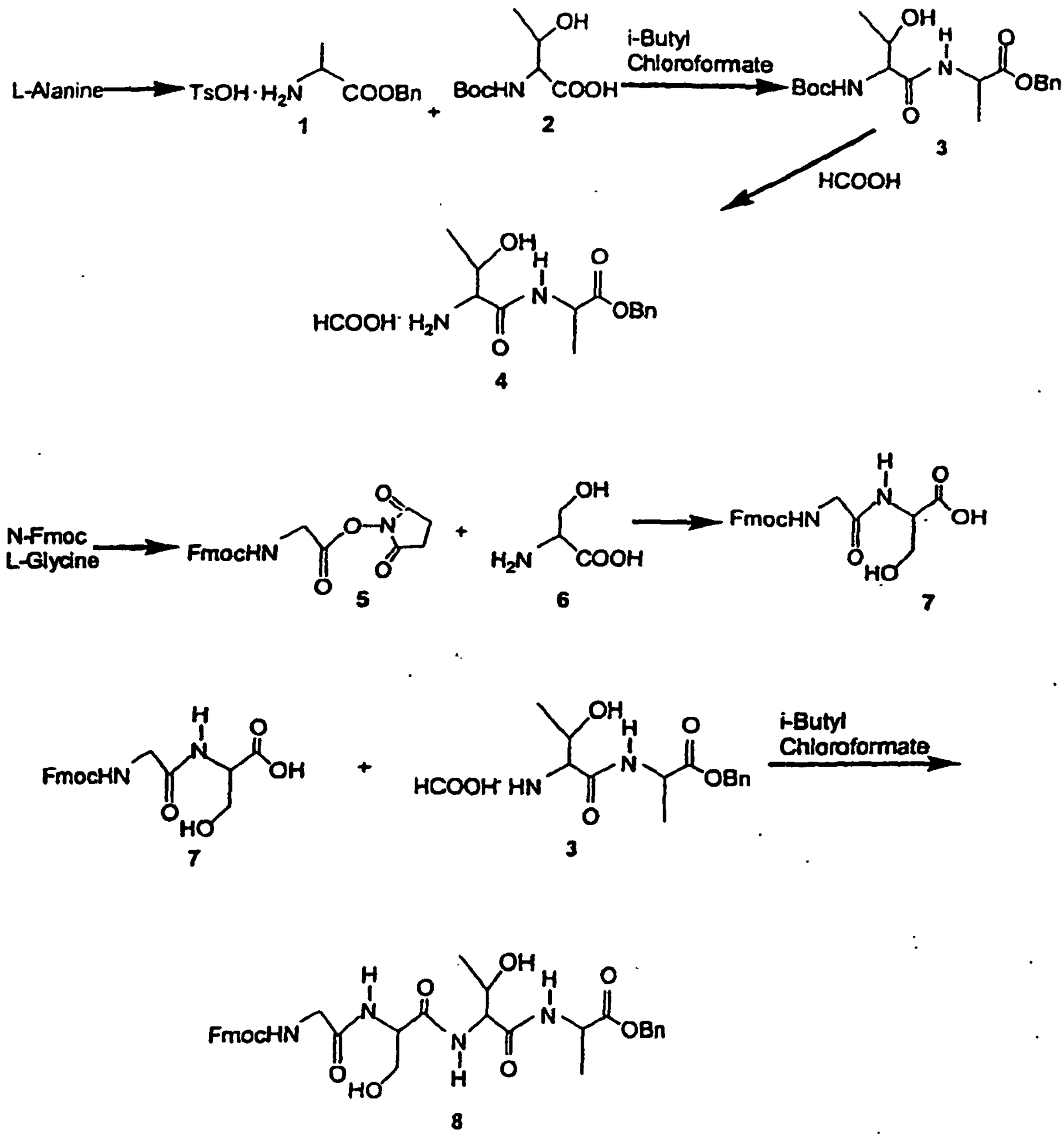


Scheme I. Synthesis of protected Peptide

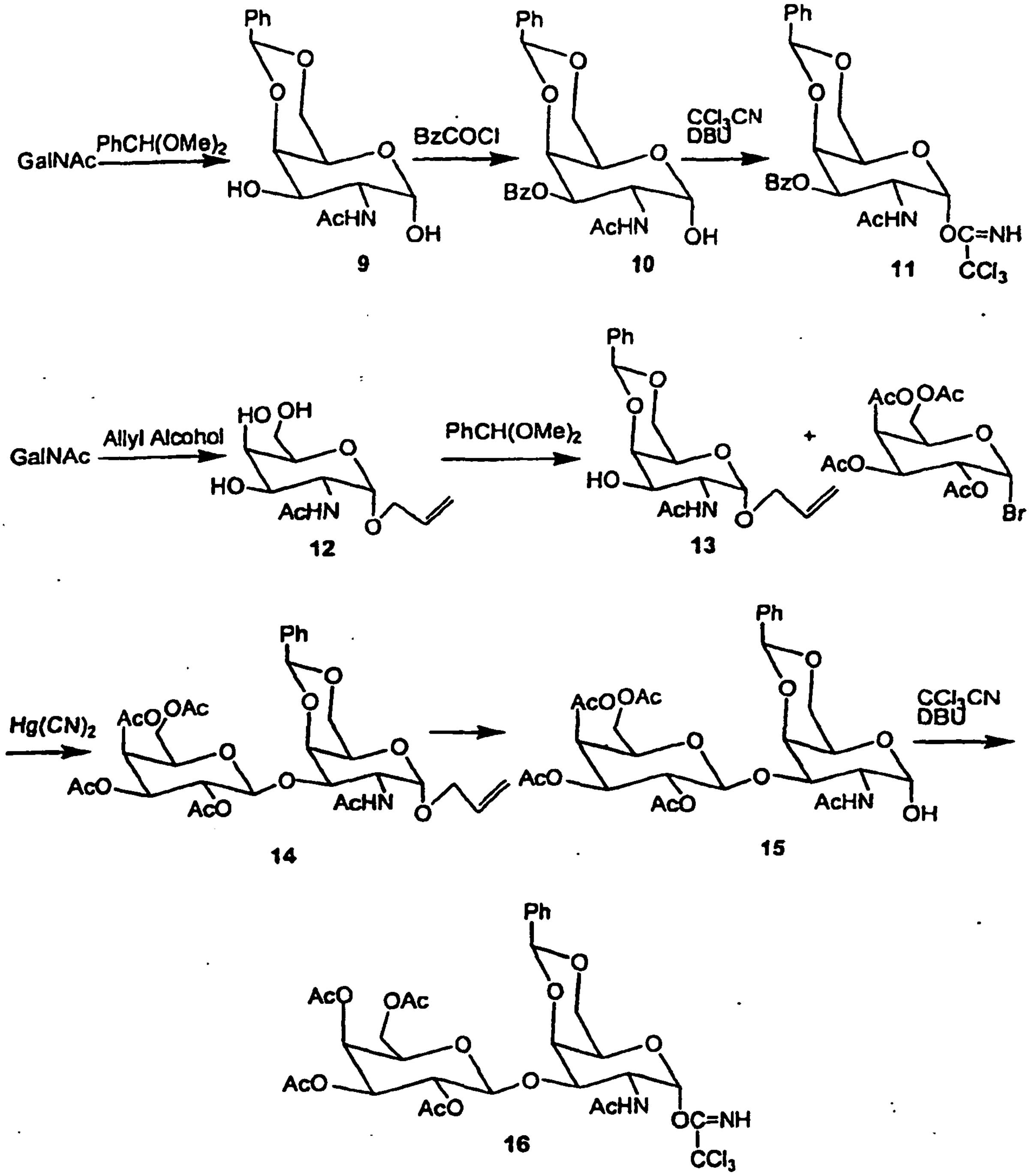


9 10 11 12 13 14 15 16

Scheme II. Synthesis of donors for first level of the library

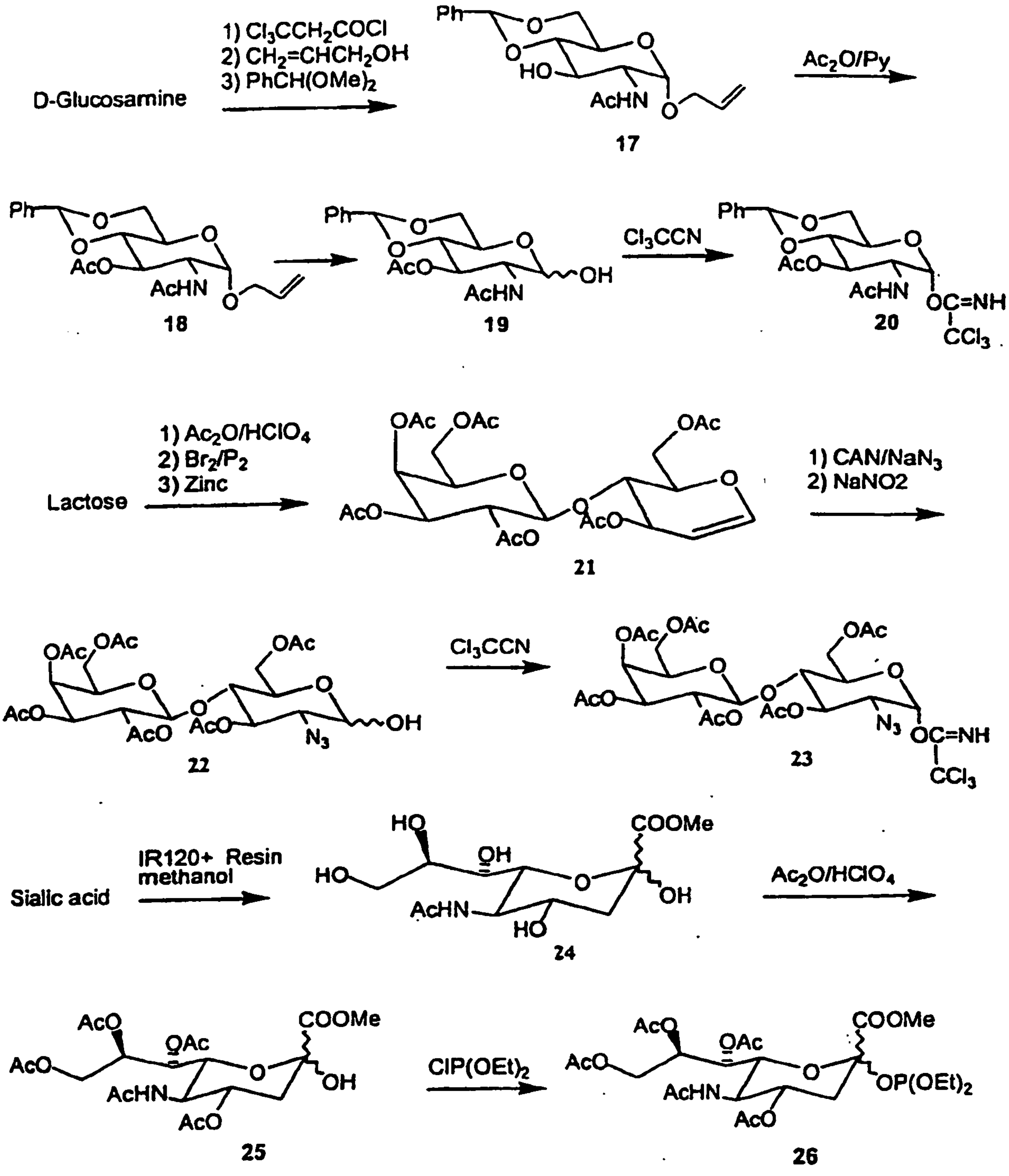


Scheme III. Synthesis of donors for second level of the library

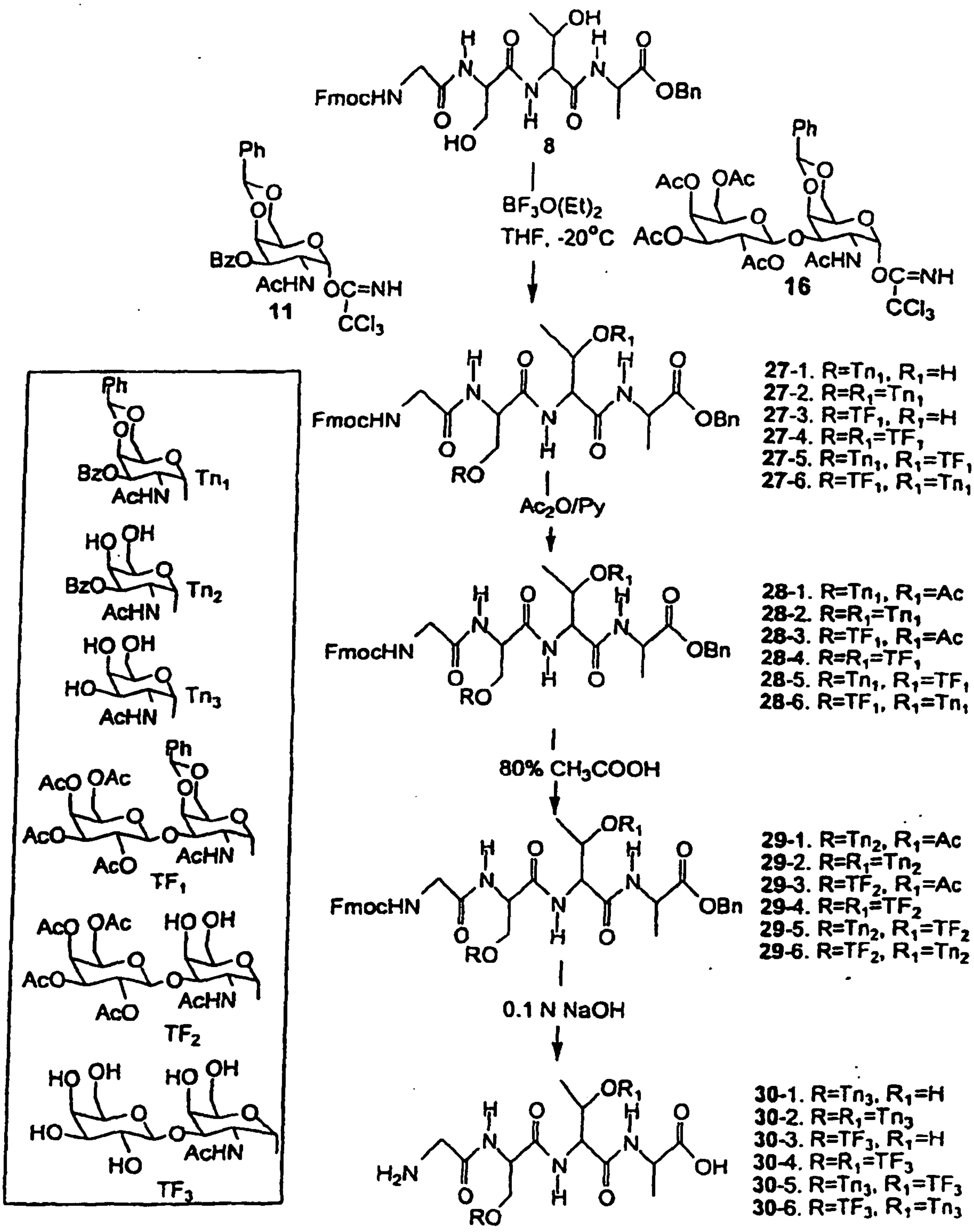


8

11

16

27-1. R=Tn$_1$, R$_1$=H
27-2. R=R$_1$=Tn$_1$
27-3. R=TF$_1$, R$_1$=H
27-4. R=R$_1$=TF$_1$
27-5. R=Tn$_1$, R$_1$=TF$_1$
27-6. R=TF$_1$, R$_1$=Tn$_1$

28-1. R=Tn$_1$, R$_1$=Ac
28-2. R=R$_1$=Tn$_1$
28-3. R=TF$_1$, R$_1$=Ac
28-4. R=R$_1$=TF$_1$
28-5. R=Tn$_1$, R$_1$=TF$_1$
28-6. R=TF$_1$, R$_1$=Tn$_1$

29-1. R=Tn$_2$, R$_1$=Ac
29-2. R=R$_1$=Tn$_2$
29-3. R=TF$_2$, R$_1$=Ac
29-4. R=R$_1$=TF$_2$
29-5. R=Tn$_2$, R$_1$=TF$_2$
29-6. R=TF$_2$, R$_1$=Tn$_2$

30-1. R=Tn$_3$, R$_1$=H
30-2. R=R$_1$=Tn$_3$
30-3. R=TF$_3$, R$_1$=H
30-4. R=R$_1$=TF$_3$
30-5. R=Tn$_3$, R$_1$=TF$_3$
30-6. R=TF$_3$, R$_1$=Tn$_3$

Scheme IV. First level of the library

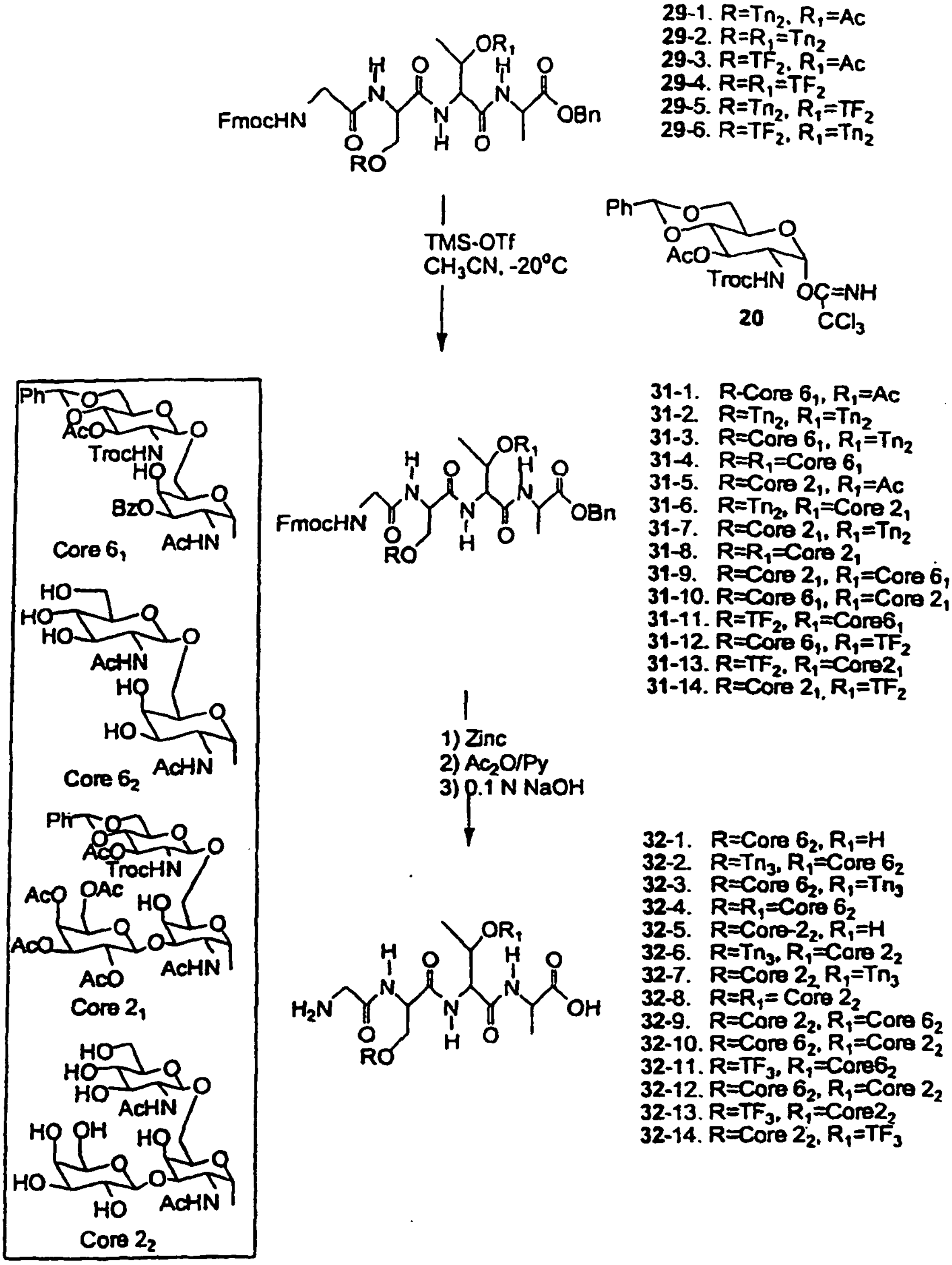


29-1. R=$Tn_2$, $R_1$=Ac
29-2. R=$R_1$=$Tn_2$
29-3. R=$TF_2$, $R_1$=Ac
29-4. R=$R_1$=$TF_2$
29-5. R=$Tn_2$, $R_1$=$TF_2$
29-6. R=$TF_2$, $R_1$=$Tn_2$

20

Core $6_1$

Core $6_2$

Core $2_1$

Core $2_2$

31-1. R-Core $6_1$, $R_1$=Ac
31-2. R=$Tn_2$, $R_1$=$Tn_2$
31-3. R=Core $6_1$, $R_1$=$Tn_2$
31-4. R=$R_1$=Core $6_1$
31-5. R=Core $2_1$, $R_1$=Ac
31-6. R=$Tn_2$, $R_1$=Core $2_1$
31-7. R=Core $2_1$, $R_1$=$Tn_2$
31-8. R=$R_1$=Core $2_1$
31-9. R=Core $2_1$, $R_1$=Core $6_1$
31-10. R=Core $6_1$, $R_1$=Core $2_1$
31-11. R=$TF_2$, $R_1$=Core$6_1$
31-12. R=Core $6_1$, $R_1$=$TF_2$
31-13. R=$TF_2$, $R_1$=Core$2_1$
31-14. R=Core $2_1$, $R_1$=$TF_2$

32-1. R=Core $6_2$, $R_1$=H
32-2. R=$Tn_3$, $R_1$=Core $6_2$
32-3. R=Core $6_2$, $R_1$=$Tn_3$
32-4. R=$R_1$=Core $6_2$
32-5. R=Core-$2_2$, $R_1$=H
32-6. R=$Tn_3$, $R_1$=Core $2_2$
32-7. R=Core $2_2$ $R_1$=$Tn_3$
32-8. R=$R_1$= Core $2_2$
32-9. R=Core $2_2$, $R_1$=Core $6_2$
32-10. R=Core $6_2$, $R_1$=Core $2_2$
32-11. R=$TF_3$, $R_1$=Core$6_2$
32-12. R=Core $6_2$, $R_1$=Core $2_2$
32-13. R=$TF_3$, $R_1$=Core$2_2$
32-14. R=Core $2_2$, $R_1$=$TF_3$

Scheme V. Second level of the library with GlcNAc as donor

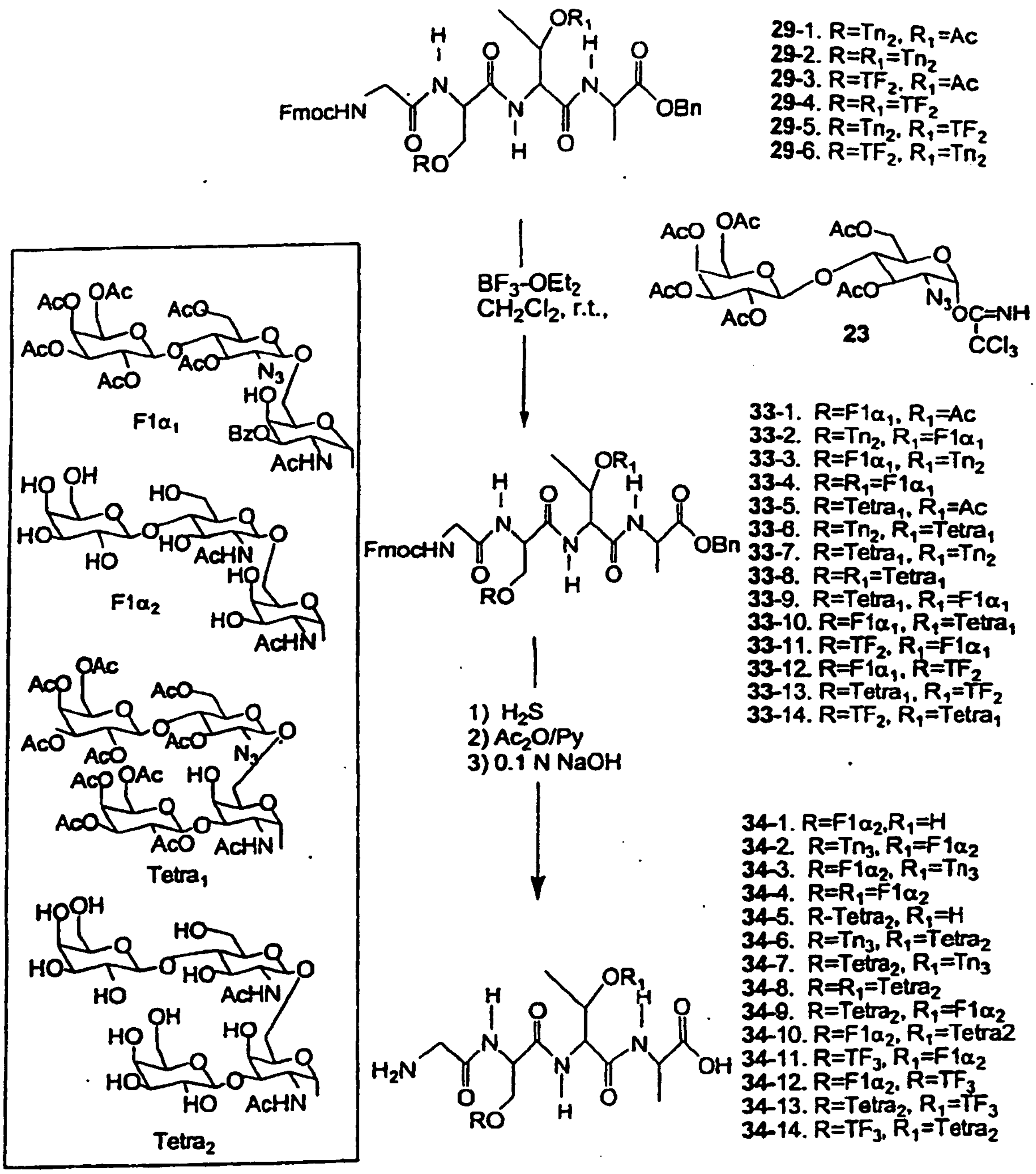


29-1. R=Tn$_2$, R$_1$=Ac
29-2. R=R$_1$=Tn$_2$
29-3. R=TF$_2$, R$_1$=Ac
29-4. R=R$_1$=TF$_2$
29-5. R=Tn$_2$, R$_1$=TF$_2$
29-6. R=TF$_2$, R$_1$=Tn$_2$

F1α$_1$

F1α$_2$

Tetra$_1$

Tetra$_2$

23

33-1. R=F1α$_1$, R$_1$=Ac
33-2. R=Tn$_2$, R$_1$=F1α$_1$
33-3. R=F1α$_1$, R$_1$=Tn$_2$
33-4. R=R$_1$=F1α$_1$
33-5. R=Tetra$_1$, R$_1$=Ac
33-6. R=Tn$_2$, R$_1$=Tetra$_1$
33-7. R=Tetra$_1$, R$_1$=Tn$_2$
33-8. R=R$_1$=Tetra$_1$
33-9. R=Tetra$_1$, R$_1$=F1α$_1$
33-10. R=F1α$_1$, R$_1$=Tetra$_1$
33-11. R=TF$_2$, R$_1$=F1α$_1$
33-12. R=F1α$_1$, R=TF$_2$
33-13. R=Tetra$_1$, R$_1$=TF$_2$
33-14. R=TF$_2$, R$_1$=Tetra$_1$

34-1. R=F1α$_2$,R$_1$=H
34-2. R=Tn$_3$, R$_1$=F1α$_2$
34-3. R=F1α$_2$, R$_1$=Tn$_3$
34-4. R=R$_1$=F1α$_2$
34-5. R-Tetra$_2$, R$_1$=H
34-6. R=Tn$_3$, R$_1$=Tetra$_2$
34-7. R=Tetra$_2$, R$_1$=Tn$_3$
34-8. R=R$_1$=Tetra$_2$
34-9. R=Tetra$_2$, R$_1$=F1α$_2$
34-10. R=F1α$_2$, R$_1$=Tetra2
34-11. R=TF$_3$, R$_1$=F1α$_2$
34-12. R=F1α$_2$, R=TF$_3$
34-13. R=Tetra$_2$, R$_1$=TF$_3$
34-14. R=TF$_3$, R$_1$=Tetra$_2$

Scheme VI. Second level of the library with N-acetyl lactosamine as donor

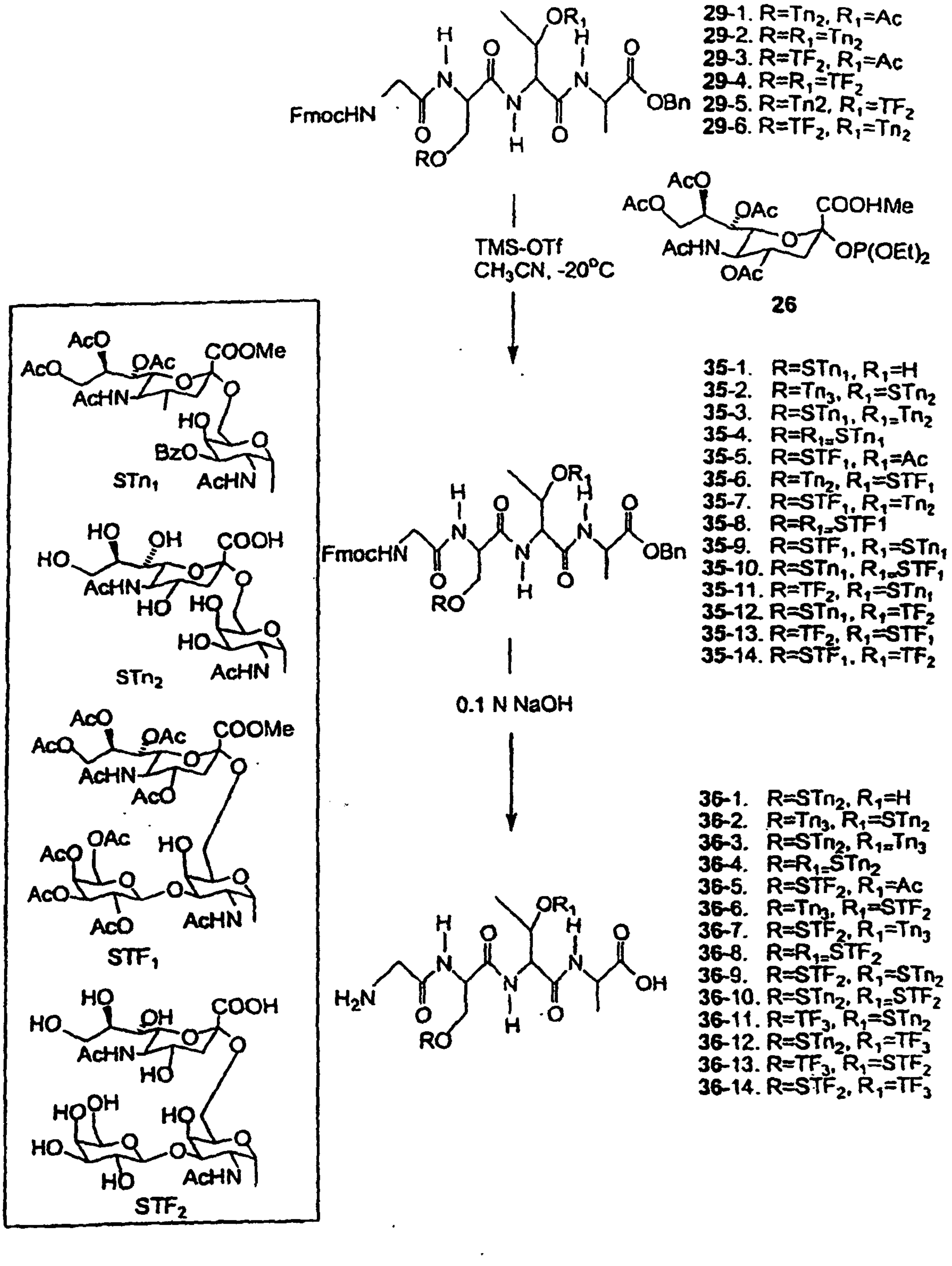


29-1. R=$Tn_2$, $R_1$=Ac
29-2. R=$R_1$=$Tn_2$
29-3. R=$TF_2$, $R_1$=Ac
29-4. R=$R_1$=$TF_2$
29-5. R=Tn2, $R_1$=$TF_2$
29-6. R=$TF_2$, $R_1$=$Tn_2$

26

$STn_1$

$STn_2$

$STF_1$

$STF_2$

35-1. R=$STn_1$, $R_1$=H
35-2. R=$Tn_3$, $R_1$=$STn_2$
35-3. R=$STn_1$, $R_1$=$Tn_2$
35-4. R=$R_1$=$STn_1$
35-5. R=$STF_1$, $R_1$=Ac
35-6. R=$Tn_2$, $R_1$=$STF_1$
35-7. R=$STF_1$, $R_1$=$Tn_2$
35-8. R=$R_1$=STF1
35-9. R=$STF_1$, $R_1$=$STn_1$
35-10. R=$STn_1$, $R_1$=$STF_1$
35-11. R=$TF_2$, $R_1$=$STn_1$
35-12. R=$STn_1$, $R_1$=$TF_2$
35-13. R=$TF_2$, $R_1$=$STF_1$
35-14. R=$STF_1$, $R_1$=$TF_2$

36-1. R=$STn_2$, $R_1$=H
36-2. R=$Tn_3$, $R_1$=$STn_2$
36-3. R=$STn_2$, $R_1$=$Tn_3$
36-4. R=$R_1$=$STn_2$
36-5. R=$STF_2$, $R_1$=Ac
36-6. R=$Tn_3$, $R_1$=$STF_2$
36-7. R=$STF_2$, $R_1$=$Tn_3$
36-8. R=$R_1$=$STF_2$
36-9. R=$STF_2$, $R_1$=$STn_2$
36-10. R=$STn_2$, $R_1$=$STF_2$
36-11. R=$TF_3$, $R_1$=$STn_2$
36-12. R=$STn_2$, $R_1$=$TF_3$
36-13. R=$TF_3$, $R_1$=$STF_2$
36-14. R=$STF_2$, $R_1$=$TF_3$

Scheme VII. Second level of the library with sialic acid as donor

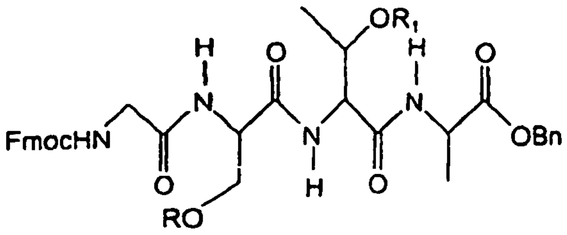

29-1. R=$Tn_2$, $R_1$=Ac
29-2. R=$R_1$=$Tn_2$
29-3. R=$TF_2$, $R_1$=Ac
29-4. R=$R_1$=$TF_2$
29-5. R=$Tn_2$, $R_1$=$TF_2$
29-6. R=$TF_2$, $R_1$=$Tn_2$

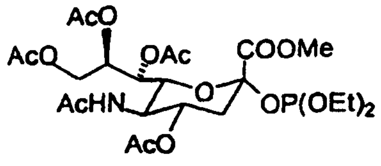

26

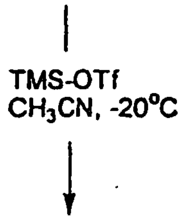


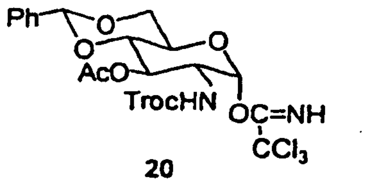

20

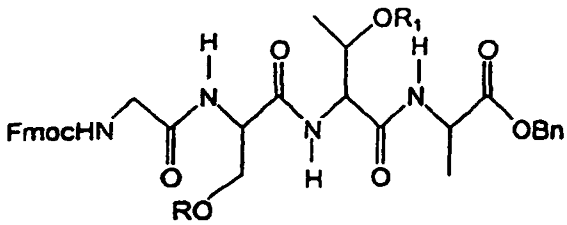

37-1. R=Core $6_1$, $R_1$=Ac
37-2. R=$Tn_2$, $R_1$=Core $6_1$
37-3. R=Core $6_1$, $R_1$=$Tn_2$
37-4. R=$R_1$=Core $6_1$
37-5. R=Core $2_1$, $R_1$=Ac
37-6. R=$Tn_2$, $R_1$=Core $2_1$
37-7. R=Core $2_1$, $R_1$=$Tn_2$
37-8. R=$R_1$=Core $2_1$
37-9. R=Core $2_1$, $R_1$=Core $6_1$
37-10. R=Core $6_1$, $R_1$=Core $2_1$
37-11. R=$STn_1$, $R_1$=Ac
37-12. R=$Tn_2$, $R_1$=$STn_1$
37-13. R=$STn_1$, $R_1$=$Tn_2$
37-14. R=$R_1$=$STn_1$
37-15. R=$TF_2$, $R_1$=Core $6_1$
37-16. R=Core $6_1$, $R_1$=$TF_2$
37-17. R=$TF_2$, $R_1$=Core $2_1$
37-18. R=Core $2_1$, $R_1$=$TF_2$
37-19. R=$TF_2$, $R_1$=$STn_1$
37-20. R=$STn_1$, $R_1$=$TF_2$
37-21. R=$STF_1$, $R_1$=Ac
37-22. R=$Tn_2$, $R_1$=$STF_1$
37-23. R=$STF_1$, $R_1$=$Tn_2$
37-24. R=$STF_1$, $R_1$=$TF_2$
37-25. R=$TF_2$, $R_1$=$STF_1$
37-26. R=$R_1$=$STF_1$
37-27. R=$STF_1$, $R_1$=$STn_1$
37-28. R=$STn_1$, $R_1$=$STF_1$
37-29. R=$STn_1$, $R_1$=Core $6_1$
37-30. R=Core $6_1$, $R_1$=$STn_1$
37-31. R=$STF_1$, $R_1$=Core $6_1$
37-32. R=Core $6_1$, $R_1$=$STF_1$
37-33. R=$STn_1$, $R_1$=Core $2_1$
37-34. R=Core $2_1$, $R_1$=$STn_1$
37-35. R=$STF_1$, $R_1$=Core $2_1$
37-36. R=Core $2_1$, $R_1$= $STF_1$

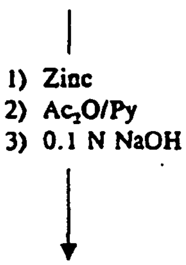

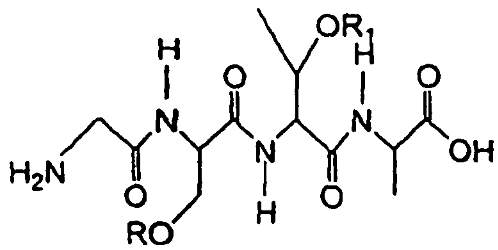

38-1. R=Core $6_2$, $R_1$=H
38-2. R=$Tn_3$, $R_1$=Core $6_2$
38-3. R=Core $6_2$, $R_1$=$Tn_3$
38-4. R=$R_1$=Core $6_2$
38-5. R=Core $2_2$, $R_1$=H
38-6. R=$Tn_3$, $R_1$=Core $2_2$
38-7. R=Core $2_2$, $R_1$=$Tn_3$
38-8. R=$R_1$=Core $2_2$
38-9. R=Core $2_2$, $R_1$=Core $6_2$
38-10. R=Core $6_2$, $R_1$= Core $2_2$
38-11. R=$STn_2$, $R_1$=H
38-12. R=$Tn_3$, $R_1$=$STn_2$
38-13. R=$STn_2$, $R_1$=$Tn_3$
38-14. R=$R_1$=$STn_2$
38-15. R=$TF_3$, $R_1$=Core $6_2$
38-16. R=Core $6_2$, $R_1$=$TF_3$
38-17. R=$TF_3$, $R_1$=Core $2_2$
38-18. R=Core $2_2$, $R_1$=$TF_3$
38-19. R=$TF_3$, $R_1$= $STn_2$
38-20. R=$STn_2$,$R_1$=$TF_3$
38-21. R=$STF_2$, $R_1$=H
38-22. R=$Tn_3$, $R_1$=$STF_2$
38-23. R=$STF_2$, $R_1$=$Tn_3$
38-24. R=$STF_2$, $R_1$=$TF_3$
38-25. R=$TF_3$, $R_1$=$STF_2$
38-26. R=$R_1$= $STF_2$
38-27. R=$STF_2$, $R_1$=$STn_2$
38-28. R=$STn_2$, $R_1$=$STF_2$
38-29. R=$STn_2$, $R_1$=Core $6_2$
38-30. R=Core $6_2$, $R_1$=$STn_2$
38-31. R=$STF_2$, $R_1$=Core $6_2$
38-32. R=Core $6_2$, $R_1$=$STF_2$
38-33. R=$STn_2$, $R_1$=Core $2_2$
38-34. R=Core $2_2$, $R_1$=$STn_2$
38-35. R=$STF_2$, $R_1$=Core $2_2$
38-36. R=Core $2_2$, $R_1$=$STF_2$

**Scheme VIII. Second level of the library with GlcNAc and sialic acid as donors**

## Claims

1. A method of generating a library of peptides or peptide-like structures that have at least one glycosylation site, comprising the steps of:

    a) randomly glycosylating a core having at least one glycosylation site with at least one glycosyl donor, optionally blocking unreacted gylcosylation sites on the glycosylated cores and optionally selectively removing one or more protecting groups on the carbohydrate groups introduced at the first level; whereby a first level library of glycosylated core is created; and then

    b) optionally randomly glycosylating said first level library of glycosylated cores, or a combination of first level

libraries of glycosylated core, with at least one glycosyl donor, and optionally selectively removing one or more designated protecting groups on the carbohydrate groups introduced at the second level; whereby a second level library of glycosylated cores is created.

**2.** A method according to claim 1, which further comprises further randomly glycosylating said second level library of glycosylated cores, or a combination of second level or first and second level libraries of glycosylated cores, with at least one glycosyl donor, and optionally selectively removing one or more designated protecting groups on the carbohydrate groups introduced at the third level; whereby a third level library of glycosylated cores is created; and optionally repeating the foregoing step to produce fourth and higher level libraries of increased diversity.

**3.** A method according to claim 2, wherein said peptide has an amino acid sequence GVTSAPDTRPAPGSTA.

**4.** A method according to claim 2, wherein said peptide has an amino acid sequence GSTA.

**5.** A method according to claim 2, wherein said unreacted glycosylation sites are blocked.

**6.** A method according to claim 5, wherein said sited are blocked by acetylation.

**7.** A method according to claim 3, wherein said glycosyl donors are selected from the group consisting of GalNAc, βGal (1-3) αGalNAc and sialyl.

**8.** A method according to claim 4, wherein said glycosyl donors are selected from the group consisting of GalNAc, βGal (1-3) αGalNAc and sialyl.

**9.** A method according to claim 1, wherein hydroxyl groups on said glycosyl donors are protected prior to reaction of said glycosyl donors with said core or said glycosylated core.

**10.** A method according to claim 9, wherein said hydroxyl groups are deprotected after reaction with said core or said glycosylated core.

**11.** A method according to claim 10, wherein some of said hydroxyl groups are removed during said deprotection step.

**12.** A method according to claim 1, wherein said core is a peptide.

**13.** A method according to claim 1, wherein said core does not contain peptide linkages.

**14.** A method according to claim 1, wherein said core comprises natural glycosylation sites.

**15.** A method according to claim 1, wherein said core comprises unnatural glycosylation sites.

**16.** A method according to claim 1, wherein said core comprises tandem repeats.

**17.** A method according to claim 1, wherein each glycosylation site on said core is unique and distinguishable from other sites due to distinct structural features in the vicinity of the site.

**18.** A method according to claim 1, wherein said core is a hybrid core comprising a non-peptide polymer to which natural amino acid side chains with natural amino acid side chains with natural glycosylation sites are attached.

**19.** A method according to claim 1, wherein said glycosylation sites provide hydroxy functions for O-glycosylation or carboxy or carboxamido functional groups for N-glycosylation.

**20.** A method according to claim 1, wherein said glycosylation sites include one or more of serine, threonine, hydroxylysine and asparagine.

**21.** A method according to claim 1, wherein said glycosylation sites consist entirely of d-optical configuration.

**22.** A method according to claim 1, wherein said core is constructed entirely of d-amino acids.

**23.** A method according to claim 1, wherein said core is linear.

**24.** A method according to claim 1, wherein said core is cyclic.

**25.** A method according to claim 1, wherein said core comprises a UV-active or fluorescent label.

**26.** A method according to claim 1, wherein said core comprises hydrophobic amino acids which increase the solubility of the core in organic solvents.

**27.** A method according to claim 1, wherein said glycosylation sites are spaced, singly or in clusters, between sequences that include hydrophobic amino acids.

**28.** A method according to claim 1, wherein lipid chains are incorporated into said core.

**29.** A method according to claim 1, wherein said glycosyl donors are unnatural.

**30.** A method according to claim 1, wherein said glycosyl donors comprise structures associated with adhesion ligands for bacterial receptors that are expressed on human cell surface antigens.

**31.** A method according to claim 1, wherein said glycosyl donors comprise structures associated with malignant cell antigens.

**32.** A method of identifying a biologically-active compound, comprising:

generating a library of glycosylated cores produced by the method of claim 1; and
screening components of said library for drug-like, competitive inhibitory, immunostimulatory or antibody-like activity.

**33.** A method of identifying an anti-viral compound, comprising:

generating a library of glycosylated cores produced by the method of claim 1; and
screening components of said library for anti-viral activity.

**34.** A method of identifying an anti-bacterial compound, comprising:

generating a library of glycosylated cores produced by the method of claim 1;
wherein said glycosyl donors comprise structures associated with adhesion ligands for bacterial receptors that are expressed on human cell surface antigens; and
screening components of said library for the ability competitively to inhibit bacterial adhesion to a host cell.

**35.** A method of identifying compounds for detection or treatment of cancer, comprising:

generating a library of glycosylated cores produced by the method of claim 1, and;
screening components of said library for anti-cancer activity.

**Patentansprüche**

**1.** Verfahren zum Erzeugen einer Bibliothek von Peptiden oder peptidähnlichen Strukturen, die wenigstens eine Glycosylierungsstelle haben, welches die folgenden Stufen umfaßt:

a) zufälliges Glycosylieren eines Kerns, der wenigstens eine Glycosylierungsstelle aufweist, mit wenigstens einem Glycosyl-Donor, optional Blockieren nicht umgesetzter Glycosylierungsstellen auf den glycosylierten Kernen und optional selektives Entfernen einer oder mehrerer Schutzgruppen auf den Kohlenhydratgruppen, die auf der ersten Ebene eingeführt wurden, wobei eine Bibliothek von glycosylierten Kernen der ersten Ebene erzeugt wird, und anschließend
b) optional zufälliges Glycosylieren der Bibliothek von glycosylierten Kernen der ersten Ebene oder einer Kombination von Bibliotheken von glycosylierten Kernen der ersten Ebene mit wenigstens einem Glycosyl-Donor,

und optional selektives Entfernen einer oder mehrerer ausgewiesener Schutzgruppen auf den Kohlenhydratgruppen, die auf der zweiten Ebene eingeführt wurden, wobei eine Bibliothek von glycosylierten Kernen der zweiten Ebene erzeugt wird.

2. Verfahren nach Anspruch 1, welches weiterhin folgendes umfaßt: zufälliges Glycosylieren der Bibliothek von glycosylierten Kernen der zweiten Ebene oder einer Kombination von Bibliotheken von glycosylierten Kernen der zweiten Ebene oder der ersten und der zweiten Ebene mit wenigstens einem Glycosyl-Donor, und optional selektives Entfernen einer oder mehrerer ausgewiesener Schutzgruppen auf den Kohlenhydratgruppen, die auf der dritten Ebene eingeführt wurden, wobei eine Bibliothek von glycosylierten Kernen der dritten Ebene erzeugt wird, und optional Wiederholen der vorangegangenen Stufe, um Bibliotheken der vierten Ebene und höherer Ebenen mit zunehmender Vielfalt zu erzeugen.

3. Verfahren nach Anspruch 2, wobei das Peptid eine Aminosäuresequenz GVTSAPDTRPAPGSTA hat.

4. Verfahren nach Anspruch 2, wobei das Peptid eine Aminosäuresequenz GSTA hat.

5. Verfahren nach Anspruch 2, wobei die nicht-umgesetzten Glycosylierungsstellen blockiert werden.

6. Verfahren nach Anspruch 5, wobei die Stellen durch Acetylierung blockiert werden.

7. Verfahren nach Anspruch 3, wobei die Glycosyl-Donoren aus der Gruppe ausgewählt sind, bestehend aus GalNAc, βGal (1-3) αGalNAc und Sialyl.

8. Verfahren nach Anspruch 4, wobei die Glycosyl-Donoren aus der Gruppe ausgewählt sind, bestehend aus GalNAc, βGal (1-3) αGalNAc und Sialyl.

9. Verfahren nach Anspruch 1, wobei Hydroxylgruppen auf den Glycosyl-Donoren vor der Reaktion der Glycosyl-Donoren mit dem Kern oder dem glycosylierten Kern geschützt werden.

10. Verfahren nach Anspruch 9, wobei die Hydroxylgruppen nach der Reaktion mit dem Kern oder dem glycosylierten Kern entschützt werden.

11. Verfahren nach Anspruch 10, wobei einige der Hydroxylgruppen während der Stufe der Schutzgruppenentfernung bzw. Entschützung entfernt werden.

12. Verfahren nach Anspruch 1, wobei der Kern ein Peptid ist.

13. Verfahren nach Anspruch 1, wobei der Kern keine Peptidbindungen enthält.

14. Verfahren nach Anspruch 1, wobei der Kern natürliche Glycosylierungsstellen enthält.

15. Verfahren nach Anspruch 1, wobei der Kern nicht natürliche Glycosylierungsstellen enthält.

16. Verfahren nach Anspruch 1, wobei der Kern Tandem-Wiederholungen enthält.

17. Verfahren nach Anspruch 1, wobei jede Glycosylierungsstelle auf dem Kern einzigartig und aufgrund unterschiedlicher struktureller Merkmale in der Umgebung der Stelle von anderen Stellen unterscheidbar ist.

18. Verfahren nach Anspruch 1, wobei der Kern ein Hybridkern ist, der ein Nicht-Peptid-Polymer umfaßt, an welches natürliche Aminosäureseitenketten mit natürlichen Aminosäureseitenketten mit natürlichen Glycosylierungsstellen angehängt sind.

19. Verfahren nach Anspruch 1, wobei die Glycosylierungsstellen Hydroxyfunktionen für die O-Glycosylierung oder carboxy- oder carboxamidofunktionale Gruppen für die N-Glycosylierung bereitstellen.

20. Verfahren nach Anspruch 1, wobei die Glycosylierungsstellen eines oder mehrere von Serin, Threonin, Hydroxylysin und Asparagin beinhalten.

**21.** Verfahren nach Anspruch 1, wobei die Glycosylierungsstellen vollständig aus einer d-optischen Konfiguration bestehen.

**22.** Verfahren nach Anspruch 1, wobei der Kern vollständig aus d-Aminosäuren besteht.

**23.** Verfahren nach Anspruch 1, wobei der Kern linear ist.

**24.** Verfahren nach Anspruch 1, wobei der Kern zyklisch ist.

**25.** Verfahren nach Anspruch 1, wobei der Kern eine UV-aktive oder fluoreszente Markierung enthält.

**26.** Verfahren nach Anspruch 1, wobei der Kern hydrophobe Aminosäuren enthält, die die Löslichkeit des Kerns in organischen Lösungsmitteln steigern.

**27.** Verfahren nach Anspruch 1, wobei die Glycosylierungsstellen einzeln oder in Clustern zwischen Sequenzen, die hydrophobe Aminosäuren beinhalten, angeordnet sind.

**28.** Verfahren nach Anspruch 1, wobei Lipidketten in den Kern aufgenommen sind.

**29.** Verfahren nach Anspruch 1, wobei die Glycosyl-Donoren nicht natürlich sind.

**30.** Verfahren nach Anspruch 1, wobei die Glycosyl-Donoren Strukturen umfassen, die mit Adhäsionsliganden für Bakterienrezeptoren, die auf humanen Zelloberflächenantigenen exprimiert sind, assoziiert sind.

**31.** Verfahren nach Anspruch 1, wobei die Glycosyl-Donoren Strukturen umfassen, die mit Antigenen gegen maligne Zellen assoziiert sind.

**32.** Verfahren zum Identifizieren einer biologisch wirksamen Verbindung, welches folgendes umfaßt:

Erzeugen einer Bibliothek von glycosylierten Kernen, hergestellt durch das Verfahren von Anspruch 1, und
Screenen von Komponenten der Bibliothek auf arzneimittelartige, kompetitiv hemmende, immunstimulierende oder antikörperartige Wirkung.

**33.** Verfahren zum Identifizieren einer antiviralen Verbindung, welches folgendes umfaßt:

Erzeugen einer Bibliothek von glycosylierten Kernen, hergestellt durch das Verfahren von Anspruch 1, und
Screenen von Komponenten der Bibliothek auf antivirale Wirkung.

**34.** Verfahren zum Identifizieren einer antibakteriellen Verbindung, welches folgendes umfaßt:

Erzeugen einer Bibliothek von glycosylierten Kernen, hergestellt durch das Verfahren von Anspruch 1, wobei die Glycosyl-Donoren Strukturen umfassen, die mit Adhäsionsliganden für Bakterienrezeptoren, die auf humanen Zelloberflächenantigenen exprimiert sind, assoziiert sind, und
Screenen von Komponenten der Bibliothek auf die Fähigkeit, die Anhaftung von Bakterien an eine Wirtszelle kompetitiv zu hemmen.

**35.** Verfahren zum Identifizieren von Verbindungen für die Erkennung oder Behandlung von Krebs, welches folgendes umfaßt:

Erzeugen einer Bibliothek von glycosylierten Kernen, hergestellt durch das Verfahren von Anspruch 1, und
Screenen von Komponenten der Bibliothek auf eine Anti-Krebs-Wirkung.

## Revendications

**1.** Procédé pour engendrer une banque de peptides ou de structures de type peptidique ayant au moins un site de glycosylation, comprenant les étapes:

a) de glycosylation aléatoire d'un noyau ayant au moins un site de glycosylation avec au moins un donneur de glycosyle, facultativement de blocage des sites de glycosylation n'ayant pas réagi sur les noyaux glycosylés et facultativement d'élimination sélective d'un ou plusieurs groupes protecteurs sur les groupes glucidiques introduits au premier niveau ; ce qui permet de créer une banque de premier niveau de noyaux glycosylés ; et ensuite
b) facultativement de glycosylation aléatoire de ladite banque de premier niveau de noyaux glycosylés, ou d'une association de banques de premier niveau de noyaux glycosylés, avec au moins un donneur de glycosyle, et facultativement d'élimination sélective d'un ou plusieurs groupes protecteurs désignés sur les groupes glucidiques introduits au second niveau ; ce qui permet de créer une banque de second niveau de noyaux glycosylés.

**2.** Procédé suivant la revendication 1, qui comprend en outre la glycosylation aléatoire de ladite banque de second niveau de noyaux glycosylés, ou d'une association de banques de second niveau ou de premier et second niveaux de noyaux glycosylés, avec au moins un donneur de glycosyle, et facultativement l'élimination sélective d'un ou plusieurs groupes protecteurs désignés sur les groupes glucidiques introduits au troisième niveau ; ce qui permet de créer une banque de troisième niveau de noyaux glycosylés, et facultativement la répétition de l'étape précédente pour produire des banques de quatrième niveau et de niveaux supérieurs ayant une diversité accrue.

**3.** Procédé suivant la revendication 2, dans lequel ledit peptide a une séquence d'aminoacides GVTSAPDTRPAPGSTA.

**4.** Procédé suivant la revendication 2, dans lequel ledit peptide a une séquence d'aminoacides GSTA.

**5.** Procédé suivant la revendication 2, dans lequel lesdits sites de glycosylation n'ayant pas réagi sont bloqués.

**6.** Procédé suivant la revendication 5, dans lequel lesdits sites sont bloqués par acétylation.

**7.** Procédé suivant la revendication 3, dans lequel lesdits donneurs de glycosyle sont choisis dans le groupe consistant en GalNAc, βGal (1-3)αGalNAc et sialyle.

**8.** Procédé suivant la revendication 4, dans lequel lesdits donneurs de glycosyle sont choisis dans le groupe consistant en GalNAc, βGal (1-3)αGalNAc et sialyle.

**9.** Procédé suivant la revendication 1, dans lequel les groupes hydroxyles sur lesdits donneurs de glycosyle sont protégés avant réaction desdits donneurs de glycosyle avec ledit noyau ou ledit noyau glycosylé.

**10.** Procédé suivant la revendication 9, dans lequel lesdits groupes hydroxyle sont débarrassés de leur protection après réaction avec ledit noyau ou ledit noyau glycosylé.

**11.** Procédé suivant la revendication 10, dans lequel certains desdits groupes hydroxyles sont éliminés au cours de ladite étape de suppression de protection.

**12.** Procédé suivant la revendication 1, dans lequel ledit noyau est un peptide.

**13.** Procédé suivant la revendication 1, dans lequel ledit noyau ne contient pas de liaisons peptidiques.

**14.** Procédé suivant la revendication 1, dans lequel ledit noyau comprend des sites de glycosylation naturels.

**15.** Procédé suivant la revendication 1, dans lequel ledit noyau comprend des sites de glycosylation non naturels.

**16.** Procédé suivant la revendication 1, dans lequel ledit noyau comprend des séquences répétées en tandem.

**17.** Procédé suivant la revendication 1, dans lequel chaque site de glycosylation sur ledit noyau est unique et différenciable des autres sites en raison de caractéristiques structurales distinctes à proximité du site.

**18.** Procédé suivant la revendication 1, dans lequel ledit noyau est un noyau hybride comprenant un polymère non peptidique auquel sont fixées des chaînes latérales d'aminoacides naturels avec des chaînes latérales d'aminoacides naturels avec des sites de glycosylation naturels.

**19.** Procédé suivant la revendication 1, dans lequel lesdits sites de glycosylation fournissent des fonctions hydroxy pour

la O-glycosylation ou des groupes fonctionnels carboxy ou carboxamido pour la N-glycosylation.

**20.** Procédé suivant la revendication 1, dans lequel lesdits sites de glycosylation comprennent un ou plusieurs des résidus sérine, thréonine, hydroxylysine et asparagine.

**21.** Procédé suivant la revendication 1, dans lequel lesdits sites de glycosylation consistent intégralement en sites à configuration optique d.

**22.** Procédé suivant la revendication 1, dans lequel ledit noyau est constitué intégralement de d-aminoacides.

**23.** Procédé suivant la revendication 1, dans lequel ledit noyau est linéaire.

**24.** Procédé suivant la revendication 1, dans lequel ledit noyau est cyclique.

**25.** Procédé suivant la revendication 1, dans lequel ledit noyau comprend un marqueur actif en lumière UV ou marqueur fluorescent.

**26.** Procédé suivant la revendication 1, dans lequel ledit noyau comprend des aminoacides hydrophobes qui augmentent la solubilité du noyau dans des solvants organiques.

**27.** Procédé suivant la revendication 1, dans lequel lesdits sites de glycosylation sont espacés, isolément ou en amas, entre les séquences qui comprennent des aminoacides hydrophobes.

**28.** Procédé suivant la revendication 1, dans lequel des chaînes lipidiques sont incorporées audit noyau.

**29.** Procédé suivant la revendication 1, dans lequel lesdits donneurs de glycosyle sont non naturels.

**30.** Procédé suivant la revendication 1, dans lequel lesdits donneurs de glycosyle comprennent des structures associées à des ligands d'adhésion pour des récepteurs bactériens qui sont exprimés sur des antigènes de surface de cellules humaines.

**31.** Procédé suivant la revendication 1, dans lequel lesdits donneurs de glycosyle comprennent des structures associées à des antigènes de cellules malignes.

**32.** Procédé pour identifier un composé biologiquement actif, comprenant les étapes consistant à :

engendrer une banque de noyaux glycosylés produits par le procédé de la revendication 1 ; et
sélectionner des constituants de ladite banque pour une activité de type médicamenteux ou une activité d'inhibition compétitive, une activité immunostimulatrice ou une activité du type anticorps.

**33.** Procédé pour identifier un composé antiviral, comprenant les étapes consistant à :

engendrer une banque de noyaux glycosylés produits par le procédé de la revendication 1 ; et
sélectionner des constituants de ladite banque pour une activité antivirale.

**34.** Procédé pour identifier un composé antibactérien, comprenant les étapes consistant à :

engendrer une banque de noyaux glycosylés produits par le procédé de la revendication 1 ; lesdits donneurs de glycosyle comprenant des structures associées à des ligands d'adhésion pour des récepteurs bactériens qui sont exprimés sur des antigènes de surface de cellules humaines ; et
sélectionner des constituants de ladite banque pour l'aptitude à inhiber de manière compétitive l'adhérence bactérienne à une cellule hôte.

**35.** Procédé pour identifier des composés pour la détection ou le traitement du cancer comprenant les étapes consistant à :

engendrer une banque de noyaux glycosylés produits par le procédé de la revendication 1 ; et
sélectionner des constituants de ladite banque pour une activité anticancéreuse.

# FIGURE 1

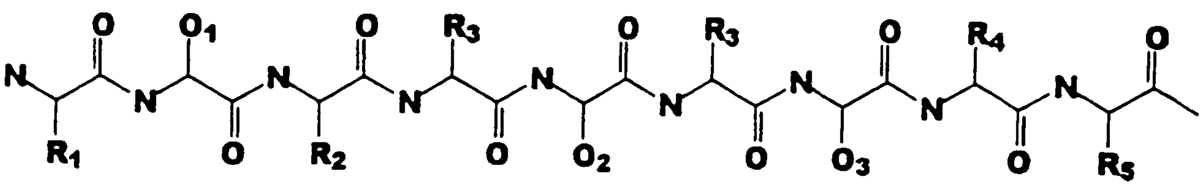

**Combinatorial glycopeptides**

**$O_1$, $O_2$, $O_3$ = Glycosylation sites**

**$R_1$ to $R_5$ = Side chains that create site specificity**

# FIGURE 2

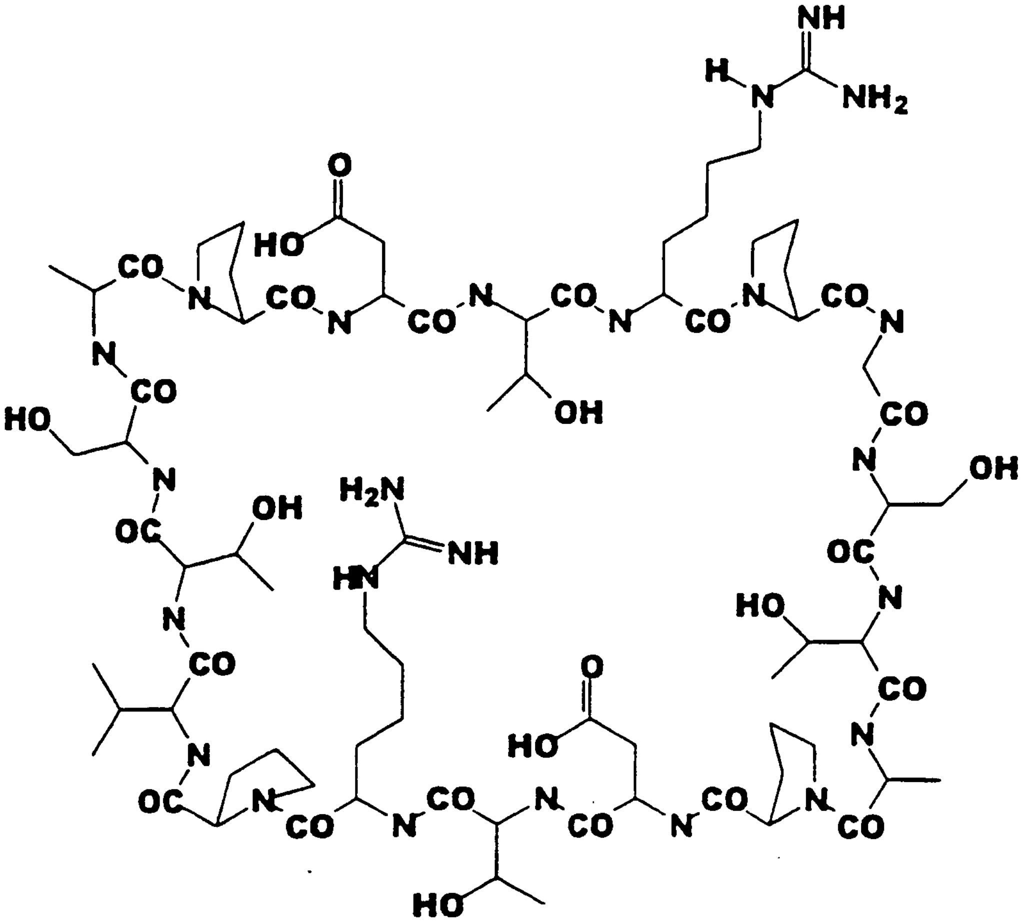

# FIGURE 3

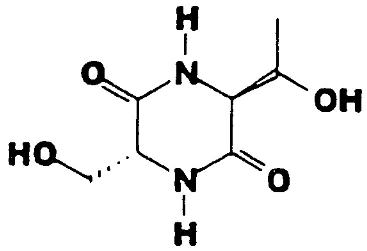

**THE SIMPLEST CYCLIC PEPTIDE**

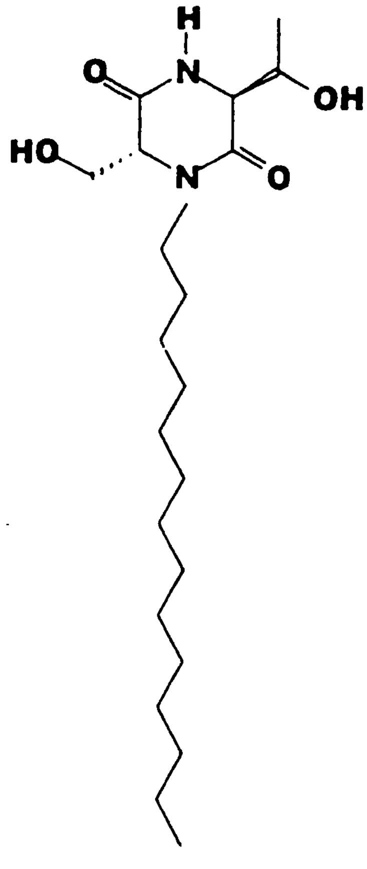

**A SOLUBLE VERSION OF THE ABOVE (with $C_{14}$ lipid)**

# FIGURE 4

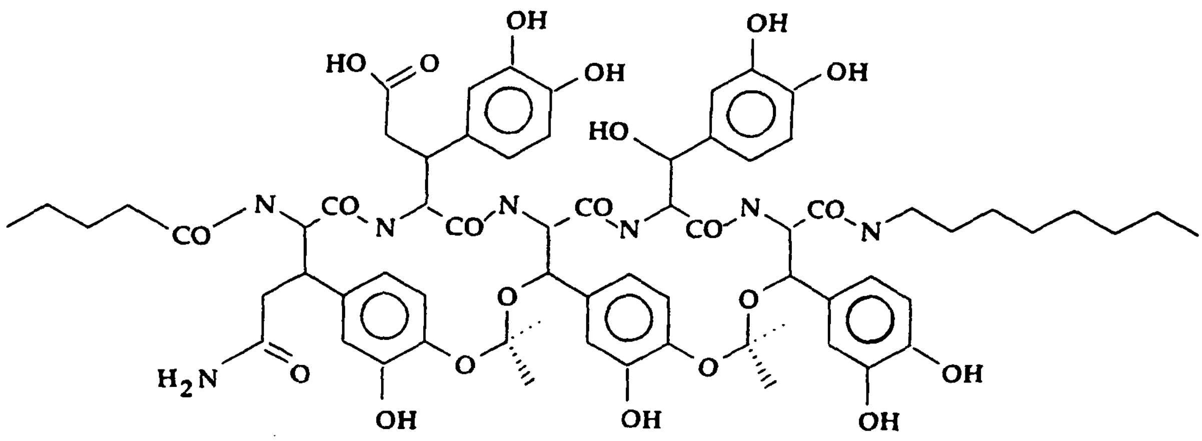

# FIGURE 5

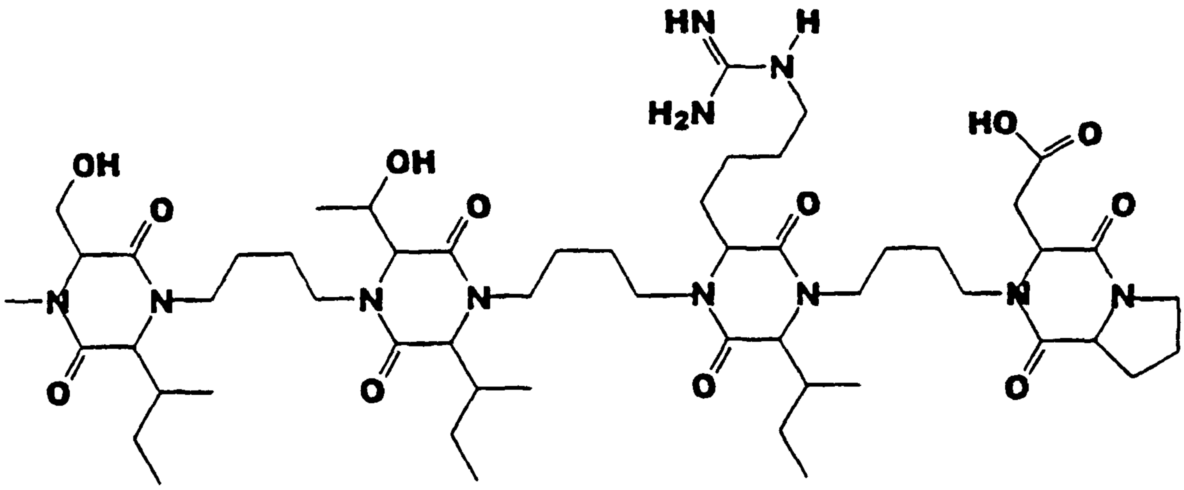

# FIGURE 6

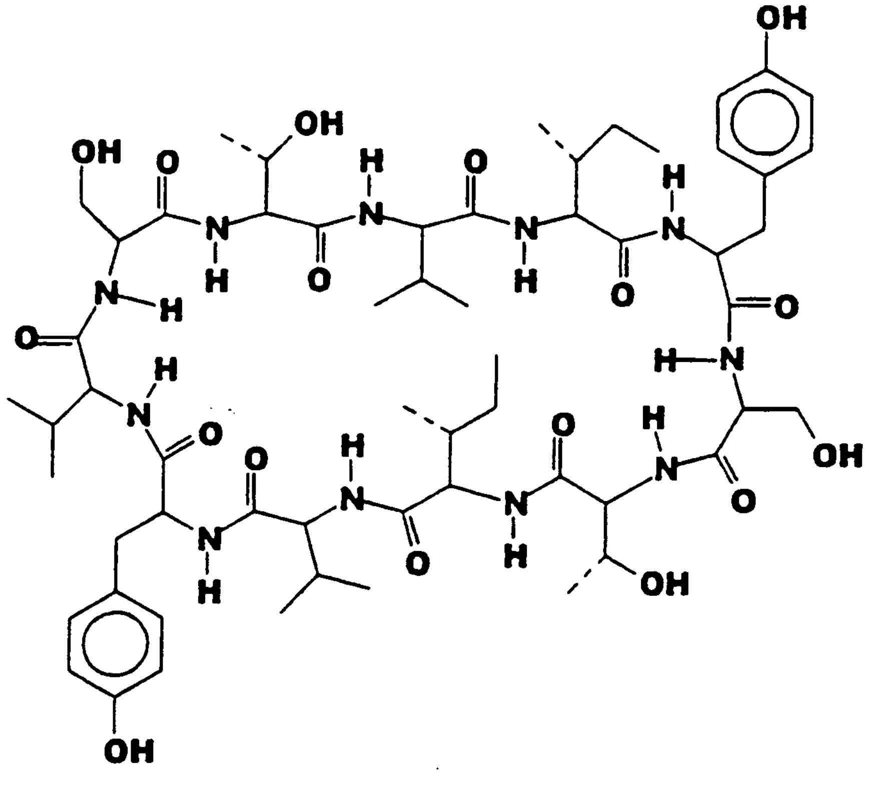

# FIGURE 7

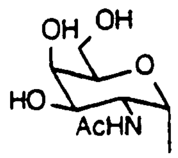

Tn

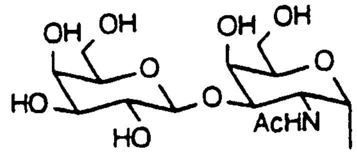

TF

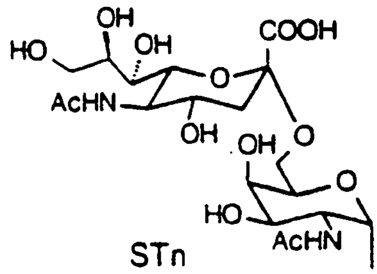

STn

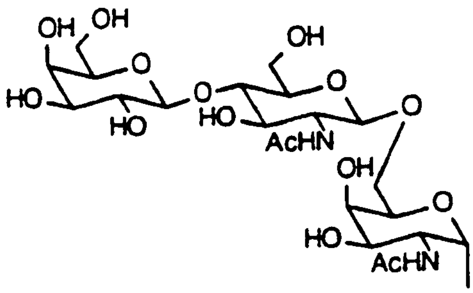

F1α

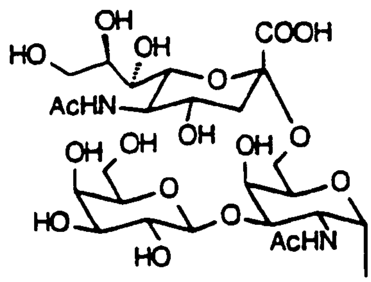

STF

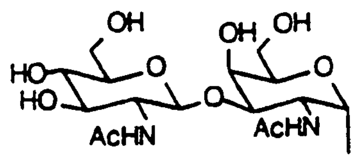

Core 3

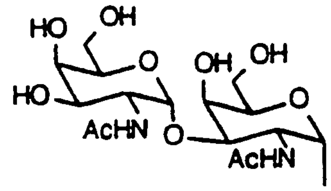

Core 5

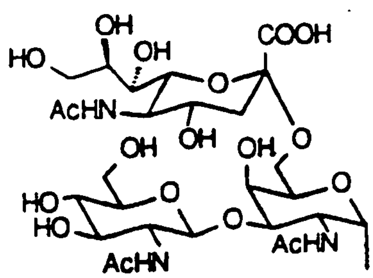

Sialyl Core 3

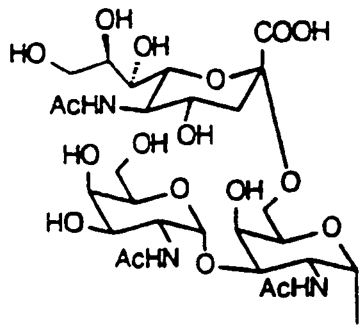

Sialyl Core 5

# FIGURE 8

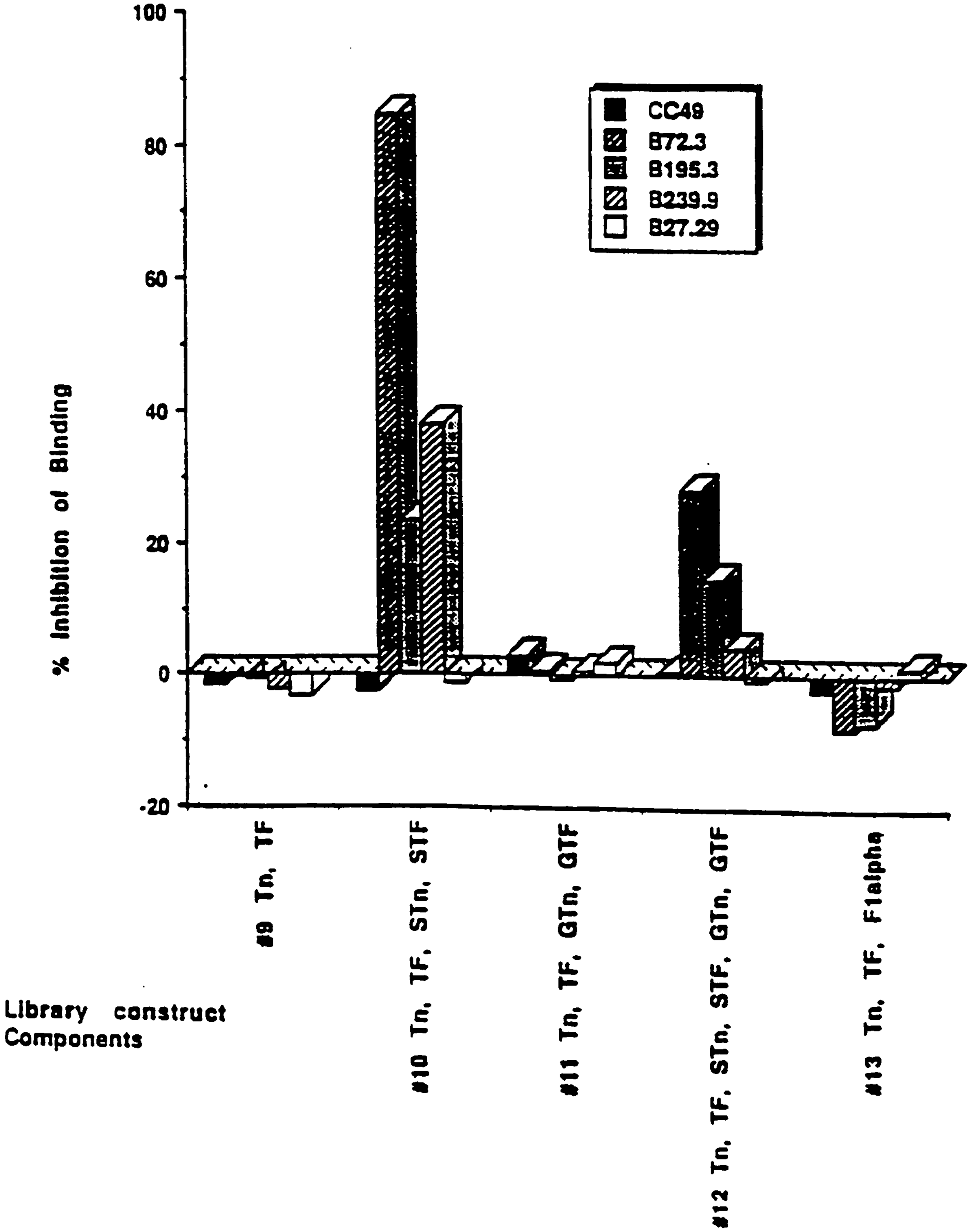
100
80
60
40
20
0
-20
% Inhibition of Binding
CC49
B72.3
B195.3
B239.9
B27.29
#9 Tn, TF
#10 Tn, TF, STn, STF
#11 Tn, TF, GTn, GTF
#12 Tn, TF, STn, STF, GTn, GTF
#13 Tn, TF, F1alpha
Library construct
Components